(19) Europäisches Patentamt
European Patent Office
Office européen des brevets

(11) EP 4 631 939 A1

(12) EUROPEAN PATENT APPLICATION
published in accordance with Art. 153(4) EPC

(43) Date of publication:
**15.10.2025 Bulletin 2025/42**

(21) Application number: **23888096.7**

(22) Date of filing: **10.11.2023**

(51) International Patent Classification (IPC):
***C07D 401/14*** *(2006.01)* ***C07D 217/00*** *(2006.01)*
***A61K 31/4725*** *(2006.01)* ***A61P 35/00*** *(2006.01)*

(52) Cooperative Patent Classification (CPC):
**A61K 31/4725; A61P 35/00; C07D 217/00; C07D 401/14**

(86) International application number:
**PCT/CN2023/130877**

(87) International publication number:
**WO 2024/099411 (16.05.2024 Gazette 2024/20)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **11.11.2022 CN 202211410146**

(71) Applicant: **CSPC Zhongqi Pharmaceutical Technology (Shijiazhuang) Co., Ltd.**
**Shijiazhuang, Hebei 050035 (CN)**

(72) Inventors:
- **TIAN, Yunfeng**
  **Shijiazhuang, Hebei 050035 (CN)**
- **CUI, Qiaoli**
  **Shijiazhuang, Hebei 050035 (CN)**
- **WU, Shasha**
  **Shijiazhuang, Hebei 050035 (CN)**
- **GUO, Feng**
  **Shijiazhuang, Hebei 050035 (CN)**
- **FENG, Lei**
  **Shijiazhuang, Hebei 050035 (CN)**
- **NIU, Miao**
  **Shijiazhuang, Hebei 050035 (CN)**
- **LIU, Feng**
  **Shijiazhuang, Hebei 050035 (CN)**
- **LI, Pengfei**
  **Shijiazhuang, Hebei 050035 (CN)**
- **ZHAO, Dong**
  **Shijiazhuang, Hebei 050035 (CN)**
- **MI, Bin**
  **Shijiazhuang, Hebei 050035 (CN)**

(74) Representative: **Gille Hrabal Partnerschaftsgesellschaft mbB Patentanwälte**
**Brucknerstraße 20**
**40593 Düsseldorf (DE)**

# (54) SALT AND CRYSTAL FORM OF ANTI-TUMOR HETEROCYCLIC COMPOUND

(57) Provided are a crystal form of a free base of compound I, a salt of compound I and a crystal form of the salt of compound (I). The crystal form and the salt form have excellent effects in terms of physical stability, chemical stability, hygroscopicity and other aspects, have a good clinical application value and can be used as excellent alternative forms for subsequent drug development.

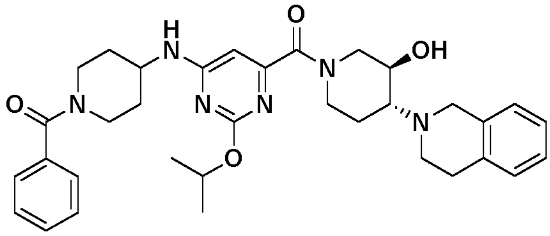

Compound I

EP 4 631 939 A1

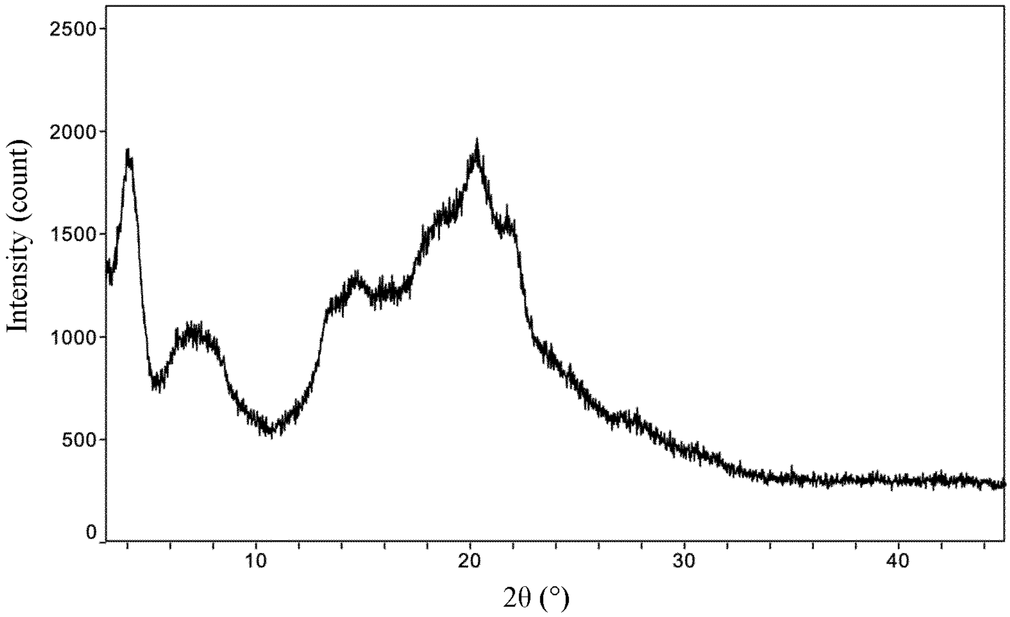


FIG. 1

## Description

CROSS-REFERENCE TO RELATED APPLICATION

The present disclosure claims priority to the Patent Application for Invention with the application No. 202211410146.4 and entitled "SALT AND CRYSTAL FORM OF ANTI-TUMOR HETEROCYCLIC COMPOUND" filed in China on November 11, 2022, the content of which is incorporated herein by reference in its entirety.

## TECHNICAL FIELD

The present disclosure relates to the technical field of crystalline pharmaceuticals, and in particular to the crystal forms , salts and preparation methods of compound (6-((1-benzoylpiperidin-4-yl)amino)-2-isopropoxypyrimidin-4-yl) ((3*R*,4*R*)-4-(3,4-dihydroisoquinolin-2(1H)-yl)-3-hydroxypiperidin-1-yl)methanone (hereinafter referred to as compound I or compound of formula I) as a PRMTS inhibitor. Furthermore, the present disclosure also relates to the use of the crystal forms and salts of compound I in the prevention and/or treatment of PRMT5-related diseases and/or disorders.

## BACKGROUND

PRMT5, an abbreviation for protein arginine N-methyltransferase 5, is one of the protein arginine methyltransferases (PRMTs). It is a novel epigenetic modification-related anti-tumor target. PRMTS has several aliases, namely Hsl7, Jbp1, Skb1, Capsuleen, and Dart5. It is the primary enzyme responsible for arginine monomethylation and symmetric dimethylation. A growing body of literature has demonstrated that PRMTs play crucial roles in diverse biological processes, such as cell growth and proliferation, apoptosis, metastasis, etc.

The function of PRMTs is to transfer a methyl group from S-adenosylmethionine (AdoMet or SAM) to arginine residues in histones or other proteins, forming methylarginine and S-adenosylhomocysteine (SAH). Currently, nine members of this family (PRMT1-9) have been identified. Based on the different modes of catalyzing arginine methylation, PRMTs can be classified into three types: Type I PRMTs include PRMT1, PRMI2, PRMT3, PRMT4, PRMT6, and PRM8, which catalyze the formation of monomethylarginine (MMA) and asymmetric dimethylarginine (aDMA); Type II PRMTs include PRMTS and PRMT9, which catalyze the formation of MMA and symmetric dimethylarginine (sDMA); Type III PRMTs are represented by PRMT7, which can only catalyze the formation of MMA. As an epigenetic enzyme, PRMTS can symmetrically methylate arginine residues of histones or non-histone substrates, influencing multiple target genes and multiple signaling pathways. PRMTS plays a crucial role in protein methylation, such as participating in alternative splicing, post-transcriptional regulation, RNA processing, cell proliferation, cell differentiation, cell apoptosis, tumorigenesis, etc. Substances that selectively inhibit PRMTS can be used as potential, powerful new anti-cancer drugs. The development of new drugs targeting PRMTS has a positive gap-filling effect on solving unmet clinical needs.

## SUMMARY

Technical Problems Solved by the Present Disclosure

Chinese Patent Application No. 202210517649.5 and International Patent Application No. PCT/CN2022/092346 disclose compounds as PRMTS inhibitors and preparation methods therefor, including compound I (the structure is shown as follows) and a preparation method therefor.

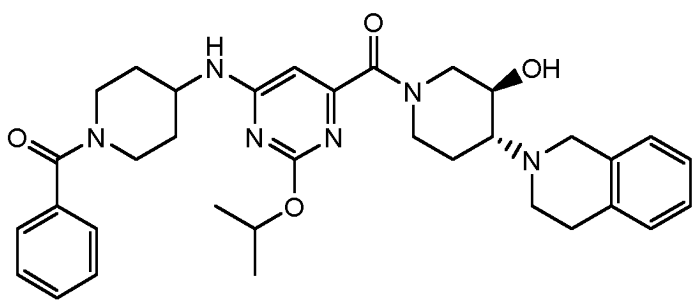

Compound I

Compound I is an effective PRMTS inhibitor. *In vitro* studies on its inhibitory activity against the enzymatic activity

have shown that it exhibits strong inhibitory effects on the PRMTS enzyme. Moreover, in an experiment of subcutaneous xenograft in female NOD/SCID mice using the human origin B-cell non-Hodgkin's lymphoma Z-138 cell strain, compound I has shown excellent tumor inhibition effects. Therefore, compound I can be used as a promising compound for preventing and/or treating PRMT5-mediated diseases.

Currently, there are no reports on the crystal forms of compound I or its salts. A comprehensive and systematic screening of polymorphs and salt forms is one of the indispensable and important research contents. Therefore, it is necessary to conduct further screening of the crystal forms of compound I and its salts and develop crystal forms or salt forms suitable for large-scale production, so as to provide more and better options for the subsequent drug development.

Technical Solutions to the problems

One of the objects of the present disclosure is to provide a crystal form A of compound I, which has characteristic peaks at 2θ angles of 4.0±0.2°, 18.4±0.2°, 20.3±0.2°, and 21.8±0.2° in an X-ray powder diffraction (XRPD) pattern using Cu-Kα radiation.

Preferably, the crystal form A has characteristic peaks at 2θ angles of 4.0±0.2°, 14.7±0.2°, 15.8±0.2°, 18.4 ±0.2°, 20.3±0.2°, and 21.8±0.2° in the XRPD pattern using Cu-Kα radiation.

More preferably, the crystal form A has characteristic peaks at 2θ angles of 4.0±0.2°, 7.1±0.2°, 13.6±0.2°, 14.7 ±0.2°, 15.8±0.2°, 18.4±0.2°, 20.3±0.2°, 21.8±0.2°, and 27.7±0.2° in the XRPD pattern using Cu-Kα radiation.

Further preferably, the crystal form A has an XRPD pattern substantially as shown in FIG. 1.

In some embodiments of the present disclosure, a differential scanning calorimetry (DSC) profile of the crystal form A has an endothermic peak at around 85.0 °C to 150.0 °C. Preferably, the DSC profile of the crystal form A has an endothermic peak at 85.0 °C ± 5 °C to 150.0 °C ± 5 °C.

More preferably, the crystal form A has a DSC profile substantially as shown in FIG. 2. In some embodiments of the present disclosure, a thermogravimetric analysis (TGA) profile of the crystal form A shows a weight loss of about 2.0% at around 100.0 °C, and also shows a weight loss of about 3.5% at around 150.0 °C.

Preferably, the TGA profile of the crystal form A shows a weight loss of about 2.0% at 100.0 °C ± 5 °C, and also shows a weight loss of about 3.5% at 150.0 °C ± 5 °C.

More preferably, the crystal form A has a TGA profile substantially as shown in FIG. 2. Another object of the present disclosure is to provide a salt of compound I, which is a hydrochloride, hydrobromide, phosphate, sulfate, methanesulfonate, p-toluenesulfonate, camphorsulfonate (e.g., L-camphorsulfonate), oxalate, maleate, tartrate (e.g., L-tartrate), fumarate, citrate, malate (e.g., L-malate), glycolate or benzoate of compound I.

Another object of the present disclosure is to provide a hydrochloride of compound I (e.g., compound I-1), wherein the salt-forming ratio (molar ratio) of compound I to hydrochloric acid is 1:2.

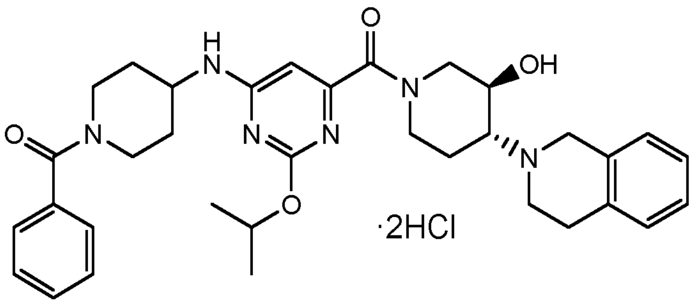

Compound I-1

Another object of the present disclosure is to provide a crystal form A of a hydrochloride of compound I, wherein the salt-forming ratio (molar ratio) of compound I to hydrochloric acid is 1:2.

In some embodiments of the present disclosure, the crystal form A of the hydrochloride of compound I has characteristic peaks at 2θ angles of 4.4±0.2°, 15.3±0.2°, 18.0±0.2°, 18.8±0.2°, and 19.3±0.2° in an XRPD pattern using Cu-Kα radiation.

Preferably, the crystal form A of the hydrochloride of compound I has characteristic peaks at 2θ angles of 4.4 ±0.2°, 11.5±0.2°, 12.7±0.2°, 15.3±0.2°, 18.0±0.2°, 18.8±0.2°, 19.3±0.2°, and 21.6±0.2° in the XRPD pattern using Cu-Kα radiation.

More preferably, the crystal form A of the hydrochloride of compound I has characteristic peaks at 2θ angles of 4.4 ±0.2°, 8.5±0.2°, 11.5±0.2°, 12.7±0.2°, 14.0±0.2°, 15.3±0.2°, 16.7±0.2°, 18.0±0.2°, 18.8±0.2°, 19.3±0.2°, 20.9 ±0.2°, 21.6±0.2°, and 23.3±0.2° in the XRPD pattern using Cu-Kα radiation.

Further preferably, the crystal form A of the hydrochloride of compound I has an XRPD pattern substantially as shown in FIG. 3.

In some embodiments of the present disclosure, a DSC profile of the crystal form A of the hydrochloride of compound I has an endothermic peak at around 149.4 °C.

Preferably, the DSC profile of the crystal form A of the hydrochloride of compound I has an endothermic peak at 149.4 °C ± 5 °C.

More preferably, the crystal form A of the hydrochloride of compound I has a DSC profile substantially as shown in FIG. 4.

In some embodiments of the present disclosure, a TGA profile of the crystal form A of the hydrochloride of compound I shows a weight loss of about 0.3% at around 100.0 °C, and also shows a weight loss of about 16.3% at around 235.0 °C.

Preferably, the TGA profile of the crystal form A of the hydrochloride of compound I shows a weight loss of about 0.3% at 100.0 °C ± 5 °C, and also shows a weight loss of about 16.3% at 235.0 °C ± 5 °C.

More preferably, the crystal form A of the hydrochloride of compound I has a TGA profile substantially as shown in FIG. 4.

Another object of the present disclosure is to provide a crystal form A of an oxalate of compound I, which has characteristic peaks at 2θ angles of 4.5±0.2°, 8.4±0.2°, 18.8±0.2°, 20.1±0.2°, and 21.2±0.2° in an XRPD pattern using Cu-Kα radiation.

Preferably, the crystal form A of the oxalate of compound I has characteristic peaks at 2θ angles of 4.5±0.2°, 6.1 ±0.2°, 8.4±0.2°, 10.4±0.2°, 16.8±0.2°, 18.8±0.2°, 20.1±0.2°, and 21.2±0.2° in the XRPD pattern using Cu-Kα radiation.

More preferably, the crystal form A of the oxalate of compound I has characteristic peaks at 2θ angles of 4.5±0.2°, 5.2±0.2°, 6.1±0.2°, 8.4±0.2°, 10.4±0.2°, 15.8±0.2°, 16.3±0.2°, 16.8±0.2°, 17.6±0.2°, 18.8±0.2°, 20.1±0.2°, 21.2 ±0.2°, 22.3±0.2°, and 23.0±0.2° in the XRPD pattern using Cu-Kα radiation.

Further preferably, the crystal form A of the oxalate of compound I has an XRPD pattern substantially as shown in FIG. 5.

In some embodiments of the present disclosure, a DSC profile of the crystal form A of the oxalate of compound I has an endothermic peak at around 157.2 °C.

Preferably, the DSC profile of the crystal form A of the oxalate of compound I has an endothermic peak at 157.2 °C ± 5 °C.

More preferably, the crystal form A of the oxalate of compound I has a DSC profile substantially as shown in FIG. 6.

In some embodiments of the present disclosure, a TGA profile of the crystal form A of the oxalate of compound I shows a weight loss of about 4.0% at around 150.0 °C.

Preferably, the TGA profile of the crystal form A of the oxalate of compound I shows a weight loss of about 4.0% at 150.0 °C ± 5 °C.

More preferably, the crystal form A of the oxalate of compound I has a TGA profile substantially as shown in FIG. 6.

Another object of the present disclosure is to provide a tartrate of compound I (e.g., *L*-tartrate, referred to as compound I-2), wherein the salt-forming ratio (molar ratio) of compound I to tartaric acid is 1:1.5.

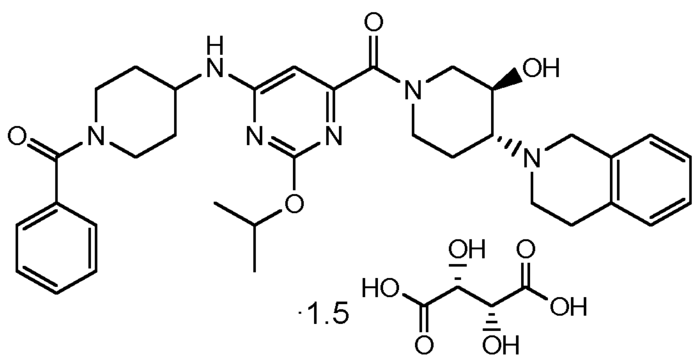

Compound I-2

Another object of the present disclosure is to provide a crystal form A of a tartrate (e.g., L-tartrate) of compound I, wherein the salt-forming ratio (molar ratio) of compound I to tartaric acid is 1:1.5.

In some embodiments of the present disclosure, the crystal form A of the tartrate of compound I has characteristic peaks at 2θ angles of 15.5±0.2°, 16.0±0.2°, 18.4±0.2°, and 20.5±0.2° in an XRPD pattern using Cu-Kα radiation.

Preferably, the crystal form A of the tartrate of compound I has characteristic peaks at 2θ angles of 5.8±0.2°, 8.8 ±0.2°, 11.7±0.2°, 15.5±0.2°, 16.0±0.2°, 18.4±0.2°, and 20.5±0.2° in the XRPD pattern using Cu-Kα radiation.

More preferably, the crystal form A of the tartrate of compound I has characteristic peaks at 2θ angles of 3.0±0.2°, 5.8±0.2°, 8.8±0.2°, 11.7±0.2°, 15.5±0.2°, 16.0±0.2°, 16.7±0.2°, 18.4±0.2°, 20.5±0.2°, and 21.2±0.2° in the XRPD pattern using Cu-Kα radiation.

Further preferably, the crystal form A of the tartrate of compound I has an XRPD pattern substantially as shown in FIG. 7.

In some embodiments of the present disclosure, a DSC profile of the crystal form A of the tartrate of compound I has an endothermic peak at around 174.8 °C.

Preferably, the DSC profile of the crystal form A of the tartrate of compound I has an endothermic peak at 174.8 °C ± 5 °C.

More preferably, the crystal form A of the tartrate of compound I has a DSC profile substantially as shown in FIG. 8.

In some embodiments of the present disclosure, a TGA profile of the crystal form A of the tartrate of compound I shows a weight loss of about 5.8% at around 150.0 °C.

Preferably, the TGA profile of the crystal form A of the tartrate of compound I shows a weight loss of about 5.8% at 150.0 °C ± 5 °C.

More preferably, the crystal form A of the tartrate of compound I has a TGA profile substantially as shown in FIG. 8.

Another object of the present disclosure is to provide a *p*-toluenesulfonate of compound I (e.g., compound I-3), wherein the salt-forming ratio (molar ratio) of compound I to *p*-toluenesulfonic acid is 1:2.

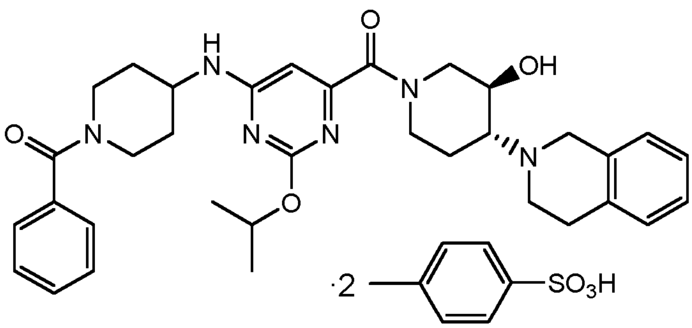

Compound I-3

Another object of the present disclosure is to provide a crystal form A of a *p*-toluenesulfonate of compound I, wherein the salt-forming ratio (molar ratio) of compound I to *p*-toluenesulfonic acid is 1:2.

In some embodiments of the present disclosure, the crystal form A of the *p*-toluenesulfonate of compound I has characteristic peaks at 2θ angles of 4.0±0.2°, 8.1±0.2°, 18.3±0.2°, and 20.0±0.2° in an XRPD pattern using Cu-Kα radiation.

Preferably, the crystal form A of the p-toluenesulfonate of compound I has characteristic peaks at 2θ angles of 4.0 ±0.2°, 8.1±0.2°, 11.7±0.2°, 12.7±0.2°, 14.6±0.2°, 18.3±0.2°, 20.0±0.2°, and 23.1±0.2° in the XRPD pattern using Cu-Kα radiation.

More preferably, the crystal form A of the p-toluenesulfonate of compound I has characteristic peaks at 2θ angles of 4.0±0.2°, 6.2±0.2°, 8.1±0.2°, 8.8±0.2°, 11.7±0.2°, 12.7±0.2°, 14.6±0.2°, 16.3±0.2°, 17.5±0.2°, 18.3±0.2°, 19.0 ±0.2°, 20.0±0.2°, and 23.1±0.2° in the XRPD pattern using Cu-Kα radiation.

Further preferably, the crystal form A of the *p*-toluenesulfonate of compound I has an XRPD pattern substantially as shown in FIG. 10.

In some embodiments of the present disclosure, a DSC profile of the crystal form A of the *p*-toluenesulfonate of compound I has endothermic peaks at around 88.5 °C and around 150.1 °C.

Preferably, the DSC profile of the crystal form A of the *p*-toluenesulfonate of compound I has endothermic peaks at 88.5 °C ± 5 °C and 150.1 °C ± 5 °C.

More preferably, the crystal form A of the *p*-toluenesulfonate of compound I has a DSC profile substantially as shown in FIG. 11.

In some embodiments of the present disclosure, a TGA profile of the crystal form A of the *p*-toluenesulfonate of compound I shows a weight loss of about 5.7% at around 130.0 °C, and also shows a weight loss of about 4.7% at around 220.0 °C.

Preferably, the TGA profile of the crystal form A of the p-toluenesulfonate of compound I shows a weight loss of about 5.7% at 130.0 °C ± 5 °C, and also shows a weight loss of about 4.7% at 220.0 °C ± 5 °C.

More preferably, the crystal form A of the *p*-toluenesulfonate of compound I has a TGA profile substantially as shown in FIG. 11.

Another object of the present disclosure is to provide a crystal form B of a *p*-toluenesulfonate of compound I, wherein the salt-forming ratio (molar ratio) of compound I to *p*-toluenesulfonic acid is 1:2.

In some embodiments of the present disclosure, the crystal form B of the *p*-toluenesulfonate of compound I has characteristic peaks at 2θ angles of 4.3±0.2°, 19.1±0.2°, and 20.2±0.2° in an XRPD pattern using Cu-Kα radiation.

Preferably, the crystal form B of the p-toluenesulfonate of compound I has characteristic peaks at 2θ angles of 4.3 ±0.2°, 9.8±0.2°, 11.5±0.2°, 19.1±0.2°, and 20.2±0.2° in the XRPD pattern using Cu-Kα radiation.

More preferably, the crystal form B of the p-toluenesulfonate of compound I has characteristic peaks at 2θ angles of 4.3±0.2°, 4.9±0.2°, 9.8±0.2°, 11.5±0.2°, 17.2±0.2°, 19.1±0.2°, 20.2±0.2°, and 21.5±0.2° in the XRPD pattern using Cu-Kα radiation.

Further preferably, the crystal form B of the p-toluenesulfonate of compound I has characteristic peaks at 2θ angles of 4.3±0.2°, 4.9±0.2°, 9.0±0.2°, 9.8±0.2°, 11.5±0.2°, 17.2±0.2°, 19.1±0.2°, 20.2±0.2°, 21.5±0.2°, and 25.7 ±0.2° in the XRPD pattern using Cu-Kα radiation.

Still further preferably, the crystal form B of the p-toluenesulfonate of compound I has characteristic peaks at 2θ angles of 4.3±0.2°, 4.9±0.2°, 7.1±0.2°, 9.0±0.2°, 9.8±0.2°, 11.5±0.2°, 12.8±0.2°, 17.2±0.2°, 19.1±0.2°, 20.2±0.2°, 21.5±0.2°, and 25.7±0.2° in the XRPD pattern using Cu-Kα radiation.

Yet still further preferably, the crystal form B of the p-toluenesulfonate of compound I has an X-ray powder diffraction pattern substantially as shown in FIG. 13 or FIG. 15.

In some embodiments of the present disclosure, a DSC profile of the crystal form B of the *p*-toluenesulfonate of compound I has an endothermic peak at around 192.1 °C, and preferably also has an endothermic peak at around 124.8 °C.

Preferably, the DSC profile of the crystal form B of the p-toluenesulfonate of compound I has an endothermic peak at 192.1 °C ± 5 °C, and preferably also has an endothermic peak at 124.8 °C ± 5 °C.

More preferably, the crystal form B of the p-toluenesulfonate of compound I has a DSC profile substantially as shown in FIG. 16.

In some embodiments of the present disclosure, a TGA profile of the crystal form B of the *p*-toluenesulfonate of compound I shows a weight loss of about 2.0% at around 167.3 °C, and also shows a weight loss of about 5.2% at around 233.7 °C.

Preferably, the TGA profile of the crystal form B of the *p*-toluenesulfonate of compound I shows a weight loss of about 2.0% at 167.3 °C ± 5 °C, and also shows a weight loss of about 5.2% at 233.7 °C ± 5 °C.

More preferably, the crystal form B of the p-toluenesulfonate of compound I has a TGA profile substantially as shown in FIG. 16.

In some embodiments of the present disclosure, the p-toluenesulfonate of compound I having the crystal form B (e.g., the *p*-toluenesulfonate with a salt-forming ratio of 1:2) is a hydrate, preferably a monohydrate.

Another object of the present disclosure is to provide a pharmaceutical composition, which comprises the hydrochloride, hydrobromide, phosphate, sulfate, methanesulfonate, *p*-toluenesulfonate, camphorsulfonate (e.g., *L*-camphorsulfonate), oxalate, maleate, tartrate (e.g., *L*-tartrate), fumarate, citrate, malate (e.g., *L*-malate), glycolate or benzoate of compound I described above.

Another object of the present disclosure is to provide a pharmaceutical composition, which comprises the crystal form A of compound I, the crystal form A of the hydrochloride of compound I, the crystal form A of the oxalate of compound I, the crystal form A of the tartrate (e.g., *L*-tartrate) of compound I, the crystal form A of the *p*-toluenesulfonate of compound I, or the crystal form B of the p-toluenesulfonate of compound I described above.

Another object of the present disclosure is to provide the use of the hydrochloride, hydrobromide, phosphate, sulfate, methanesulfonate, *p*-toluenesulfonate, camphorsulfonate (e.g., *L*-camphorsulfonate), oxalate, maleate, tartrate (e.g., *L*-tartrate), fumarate, citrate, malate (e.g., *L*-malate), glycolate or benzoate of compound I described above, or the pharmaceutical composition comprising the salt of compound I described above, for the manufacturing of a medicament for the prevention and/or treatment of PRMT5-mediated diseases and/or disorders.

Another object of the present disclosure is to provide the use of the hydrochloride, hydrobromide, phosphate, sulfate, methanesulfonate, *p*-toluenesulfonate, camphorsulfonate (e.g., *L*-camphorsulfonate), oxalate, maleate, tartrate (e.g., *L*-tartrate), fumarate, citrate, malate (e.g., *L*-malate), glycolate or benzoate of compound I described above, or the pharmaceutical composition comprising the salt of compound I described above for the manufacturing of a medicament for the prevention and/or treatment of tumor diseases.

Preferably, the tumor diseases are solid tumors or hematological tumors, preferably malignant solid tumors or hematological tumors.

More preferably, the tumor diseases are lymphomas.

Another object of the present disclosure is to provide the use of the crystal form A of compound I, the crystal form A of the hydrochloride of compound I, the crystal form A of the oxalate of compound I, the crystal form A of the tartrate of compound I, the crystal form A of the p-toluenesulfonate of compound I, or the crystal form B of the *p*-toluenesulfonate of compound I described above, or the pharmaceutical composition comprising the salt-based crystal form of compound I described above, for the manufacturing of a medicament for the prevention and/or treatment of PRMT5-mediated diseases and/or disorders.

Another object of the present disclosure is to provide the use of the crystal form A of compound I, the crystal form A of the hydrochloride of compound I, the crystal form A of the oxalate of compound I, the crystal form A of the tartrate of compound I, the crystal form A of the *p*-toluenesulfonate of compound I, or the crystal form B of the *p*-toluenesulfonate of compound I described above, or the pharmaceutical composition comprising the salt-based crystal form of compound I described above, for the manufacturing of a medicament for the prevention and/or treatment of tumor diseases. Preferably, the tumor diseases are solid tumors or hematological tumors, preferably malignant solid tumors or hematological tumors.

More preferably, the tumor diseases are lymphomas.

Another object of the present disclosure is to provide the hydrochloride, hydrobromide, phosphate, sulfate, methanesulfonate, *p*-toluenesulfonate, camphorsulfonate (e.g., *L*-camphorsulfonate), oxalate, maleate, tartrate (e.g., *L*-tartrate), fumarate, citrate, malate (e.g., *L*-malate), glycolate or benzoate of compound I described above, or the pharmaceutical composition comprising the salt of compound I described above, which is used for the prevention and/or treatment of PRMT5-mediated diseases and/or disorders. Another object of the present disclosure is to provide the hydrochloride, hydrobromide, phosphate, sulfate, methanesulfonate, *p*-toluenesulfonate, camphorsulfonate (e.g., *L*-camphorsulfonate), oxalate, maleate, tartrate (e.g., *L*-tartrate), fumarate, citrate, malate (e.g., *L*-malate), glycolate or benzoate of compound I described above, or the pharmaceutical composition comprising the salt of compound I described above, which is used for the prevention and/or treatment of a tumor diseases.

Preferably, the tumor diseases are solid tumors or hematological tumors, preferably malignant solid tumors or hematological tumors.

More preferably, the tumor diseases are lymphomas.

Another object of the present disclosure is to provide the crystal form A of compound I, the crystal form A of the hydrochloride of compound I, the crystal form A of the oxalate of compound I, the crystal form A of the tartrate of compound I, the crystal form A of the p-toluenesulfonate of compound I, or the crystal form B of the p-toluenesulfonate of compound I described above, or the pharmaceutical composition comprising the salt-based crystal form of compound I described above, which is used for the prevention and/or treatment of PRMT5-mediated diseases and/or disorders.

Another object of the present disclosure is to provide the crystal form A of compound I, the crystal form A of the hydrochloride of compound I, the crystal form A of the oxalate of compound I, the crystal form A of the tartrate of compound I, the crystal form A of the p-toluenesulfonate of compound I, or the crystal form B of the p-toluenesulfonate of compound I described above, or the pharmaceutical composition comprising the salt-based crystal form of compound I described above, which is used for the prevention and/or treatment of tumor diseases.

Preferably, the tumor diseases are solid tumors or hematological tumors, preferably malignant solid tumors or hematological tumors.

More preferably, the tumor diseases are lymphomas.

Another object of the present disclosure is to provide a method for preventing and/or treating PRMT5-mediated diseases and/or disorders, which comprises the step of administering to an individual in need thereof a prophylactically and/or therapeutically effective amount of the hydrochloride, hydrobromide, phosphate, sulfate, methanesulfonate, *p*-toluenesulfonate, camphorsulfonate (e.g., *L*-camphorsulfonate), oxalate, maleate, tartrate (e.g., *L*-tartrate), fumarate, citrate, malate (e.g., L-malate), glycolate or benzoate of compound I described above, or the pharmaceutical composition comprising the salt of compound I described above.

Another object of the present disclosure is to provide a method for preventing and/or treating tumor diseases, which comprises the step of administering to an individual in need thereof a prophylactically and/or therapeutically effective amount of the hydrochloride, hydrobromide, phosphate, sulfate, methanesulfonate, p-toluenesulfonate, camphorsulfonate (e.g., *L*-camphorsulfonate), oxalate, maleate, tartrate (e.g., *L*-tartrate), fumarate, citrate, malate (e.g., *L*-malate), glycolate or benzoate of compound I described above, or the pharmaceutical composition comprising the salt of compound I described above.

Preferably, the tumor diseases are solid tumors or hematological tumors, preferably malignant solid tumors or hematological tumors.

More preferably, the tumor diseases are lymphomas.

Another object of the present disclosure is to provide a method for preventing and/or treating PRMT5-mediated diseases and/or disorders, which comprises the step of administering to an individual in need thereof a prophylactically and/or therapeutically effective amount of the crystal form A of compound I, the crystal form A of the hydrochloride of compound I, the crystal form A of the oxalate of compound I, the crystal form A of the tartrate of compound I, the crystal form

A of the p-toluenesulfonate of compound I, or the crystal form B of the p-toluenesulfonate of compound I described above, or the pharmaceutical composition comprising the salt-based crystal form of compound I described above.

Another object of the present disclosure is to provide a method for preventing and/or treating tumor diseases, which comprises the step of administering to an individual in need thereof a prophylactically and/or therapeutically effective amount of the crystal form A of compound I, the crystal form A of the hydrochloride of compound I, the crystal form A of the oxalate of compound I, the crystal form A of the tartrate of compound I, the crystal form A of the *p*-toluenesulfonate of compound I, or the crystal form B of the *p*-toluenesulfonate of compound I described above, or the pharmaceutical composition comprising the salt-based crystal form of compound I described above.

Preferably, the tumor diseases are solid tumors or hematological tumors, preferably malignant solid tumors or hematological tumors.

More preferably, the tumor diseases are lymphomas.

For the treatment of tumor diseases, the hydrochloride, hydrobromide, phosphate, sulfate, methanesulfonate, p-toluenesulfonate, camphorsulfonate (e.g., *L*-camphorsulfonate), oxalate, maleate, tartrate (e.g., *L*-tartrate), fumarate, citrate, malate (e.g., *L*-malate), glycolate or benzoate of compound I described above, or the crystal form A of compound I, the crystal form A of the hydrochloride of compound I, the crystal form A of the oxalate of compound I, the crystal form A of the tartrate of compound I, the crystal form A of the *p*-toluenesulfonate of compound I, or the crystal form B of the *p*-toluenesulfonate of compound I described above, may be co-administered with other therapeutic agents (e.g., chemotherapeutic drugs, biological therapeutic drugs, etc.) or used in combination with other treatment methods for treatment, the other treatment methods including but not limited to radiation therapy.

<u>Effects of the Present Disclosure</u>

The present disclosure for the first time provides multiple crystal forms and salt forms of compound I. Some of the crystal forms and salt forms provided by the present disclosure exhibit excellent performance in at least one aspect of physical stability, chemical stability, hygroscopicity, etc., and possess relatively good clinical application value, thus serving as excellent candidates for subsequent drug development.

## BRIEF DESCRIPTION OF THE DRAWINGS

FIG. 1 shows an XRPD pattern of the crystal form A of compound I.
FIG. 2 shows DSC and TGA profiles of the crystal form A of compound I.
FIG. 3 shows an XRPD pattern of the crystal form A of the hydrochloride of compound I.
FIG. 4 shows DSC and TGA profiles of the crystal form A of the hydrochloride of compound I.
FIG. 5 shows an XRPD pattern of the crystal form A of the oxalate of compound I.
FIG. 6 shows DSC and TGA profiles of the crystal form A of the oxalate of compound I.
FIG. 7 shows an XRPD pattern of the crystal form A of the tartrate of compound I.
FIG. 8 shows DSC and TGA profiles of the crystal form A of the tartrate of compound I.
FIG. 9 shows a $^1$H NMR spectrum of the crystal form A of the tartrate of compound I.
FIG. 10 shows an XRPD pattern of the crystal form A of the p-toluenesulfonate of compound I.
FIG. 11 shows DSC and TGA profiles of the crystal form A of the p-toluenesulfonate of compound I.
FIG. 12 shows a $^1$H NMR spectrum of the crystal form A of the p-toluenesulfonate of compound I.
FIG. 13 shows an XRPD pattern of the crystal form B of the p-toluenesulfonate of compound I.
FIG. 14 shows a $^1$H NMR spectrum of the crystal form B of the p-toluenesulfonate of compound I.
FIG. 15 shows an XRPD pattern of the crystal form B of the p-toluenesulfonate of compound I.
FIG. 16 shows DSC and TGA profiles of the crystal form B of the p-toluenesulfonate of compound I.
FIG. 17 shows an XRPD pattern of the amorphous form of compound I.

## DETAILED DESCRIPTION

The technical solutions of the present disclosure will be further described in detail with reference to the following specific examples. The following examples are merely exemplary illustrations and explanation of the present disclosure and should not be construed as limiting the protection scope of the present disclosure. All techniques implemented based on the content described above of the present disclosure are encompassed within the claimed scope of the present disclosure.

Unless otherwise stated, the starting materials and reagents used in the following examples are all commercially available products or can be prepared by using known methods.

Testing Instruments Used in the Experiments

1. Differential scanning calorimeter analyzer TA Discovery 2500 (TA, US): A sample was weighed, then placed in a DSC Tzero sample tray with a prick and heated to a final temperature at a speed of 10 °C/min with a nitrogen purging speed of 50 mL/min in the furnace.
2. Thermogravimetric analyzer TA Discovery 55 (TA, US): A sample was placed in an equilibrated open aluminum sample tray and automatically weighed in a TGA furnace. The sample was heated to a final temperature at a speed of 10 °C/min with a nitrogen purging speed of 60 mL/min at the sample and a nitrogen purging speed of 40 mL/min at the balance.
3. Simultaneous thermal analyzer NETZSCH STA449F3 (NETZSCH, GER): The test was performed according to General Chapter 0661 Thermal Analysis of the Chinese Pharmacopoeia (2015 Edition, Volume IV), with a temperature range of 26 °C-350 °C and a scanning speed of 20.0 K/min.
4. X-ray powder diffractometer Bruker D8 Advance (Bruker, GER): The test was performed with a 2θ scanning angle from 3° to 45°, a scanning step size of 0.02°, and an exposure time of 0.12 s. The light tube voltage and current for sample testing were 40 kV and 40 mA, respectively, and the sample tray was a zero background sample tray.
5. X-ray powder diffractometer Panalytical Empyrean (Panalytical, NL): The test was performed with a 2θ scanning angle from 3° to 45°, a scanning step size of 0.013°, and an exposure time of 20.4 s. The light tube voltage and current for sample testing were 45 kV and 40 mA, respectively, and the sample tray was a zero background sample tray.
6. X-ray powder diffractometer Bruker D2 PHASER X-ray diffractometer: The test was performed using a Cu target, with a tube voltage of 30 kV, a tube current of 10 mA, a 2θ scanning range of 2°-40°, a scanning speed of 0.10 s, and a step size of 0.020°.
7. Nuclear magnetic resonance analysis ($^1$H NMR): A solid sample was dissolved in deuterated dimethylsulfoxide (DMSO-$d_6$) solvent and analyzed by NMR on the Bruker AVANCE-III (Bruker, GER).
8. Ion chromatography (IC) ICS 5000 (Thermo Fisher, US), with the instrument parameters as follows:

| **Chromatographic column** | Dionex IonPacTM AS11-HC Analytical (4 × 250 mm) | |
|---|---|---|
| **Detection ion** | Chloride ion | Tartrate ion |
| **Mobile phase** | 10 mmol/L sodium hydroxide solution | 20 mmol/L sodium hydroxide solution |
| **Flow rate** | 1.2 mL/min | 1.0 mL/min |
| **Injection volume** | 50 μL | |
| **Column temperature** | 30 °C | |
| **Run time** | 20 min | |

9. Dynamic vapor sorption/desorption (DVS) analysis was conducted using DVS Intrinsic (SMS, UK): The test adopted a gradient mode with a humidity change of 50%-95%-0%-50%, and the humidity change of each gradient was 10% in the range of 0% to 90%. The gradient endpoint was determined by dm/dt. The gradient endpoint was defined as a dm/dt less than 0.002% lasting for 10 min. After completion of the test, XRPD analysis was performed on the sample to confirm whether the solid form had changed.
10. Nuclear magnetic resonance ($^1$H NMR) analysis: Bruker AVANCE NEO 400 MHz.
11. Liquid chromatography-mass spectrometry (LC-MS): WATERS ACQUITY UPLC H-Class PLUS or/and SQD2.

**Example 1:** Preparation of Compound I

Preparation of (6-((1-benzoylpiperidin-4-yl)amino)-2-isopropoxypyrimidin-4-yl)((3*R*,4*R*)-4-(3,4-dihydroisoquinolin-2(1*H*)-yl)-3-hydroxypiperidin-1-yl)methanone (compound I)

Step 1: Preparation of 6-((1-(tert-butoxycarbonyl)piperidin-4-yl)amino)-2-chloropyrimidine-4-carboxylic acid

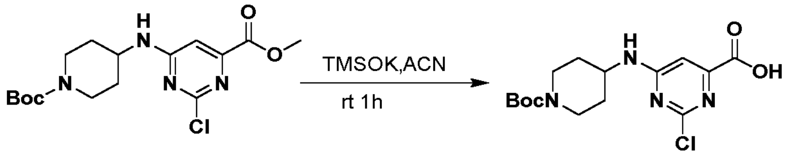


Methyl 6-((1-(*tert*-butoxycarbonyl)piperidin-4-yl)amino)-2-chloropyrimidine-4-carboxylate (500 mg, 1.347 mmol, 1.0 equiv.) was dissolved in acetonitrile (5 mL). Potassium trimethylsilanolate (0.1729 g, 1.347 mmol, 1.0 equiv.) was added, and the mixture was stirred at 25 °C for 1 h. After the reaction was completed, as detected by LC-MS, the mixture was concentrated, and the residue was dissolved in a 10% dichloromethane-methanol solution. The solution was then filtered, and the filtrate was concentrated to give the title compound (480 mg, yield: 99.8%).
LC-MS (ESI) $[M+H]^+$ = 357.2.

Step 2: Preparation of 6-((1-(tert-butoxycarbonyl)piperidin-4-yl)amino)-2-isopropoxypyrimidine-4-carboxylic acid

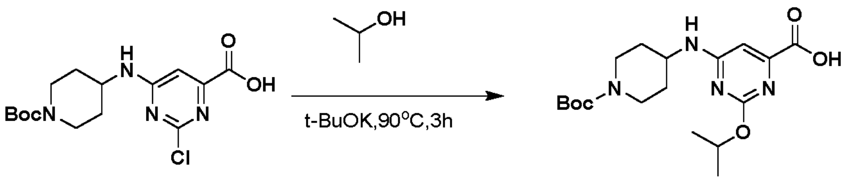


6-((1-(*tert*-Butoxycarbonyl)piperidin-4-yl)amino)-2-chloropyrimidine-4-carboxylic acid (480 mg, 1.35 mmol, 1.0 equiv.) was dissolved in isopropanol (5 mL). Potassium tert-butoxide (628.8 mg, 5.4 mmol, 4.0 equiv.) was added. The mixture was stirred at 90 °C for 3 h. After the reaction was completed, as detected by LC-MS, the mixture was concentrated, and the residue was separated and purified by preparative TLC (MeOH: DCM = 10%, v/v) to give the title compound (529 mg, yield: 99.3%).
LC-MS (ESI) $[M+H]^+$ = 381.3.

Step 3: Preparation of tert-butyl 4-((6-((3R,4R)-4-(3,4-dihydroisoquinolin-2(1H)-yl)-3-hydroxypiperidine-1-carbonyl)-2-isopropoxypyrimidin-4-yl)amino)piperidine-1-carboxylate

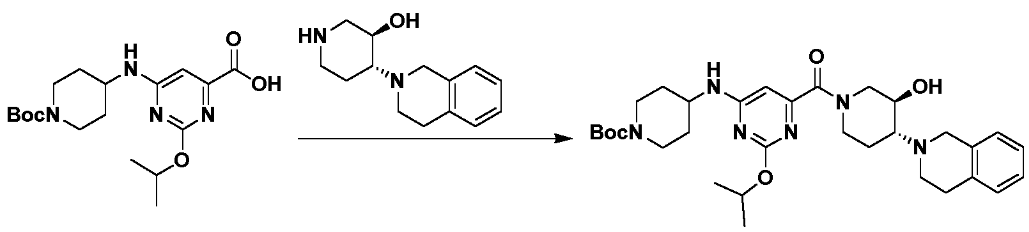

6-((1-(*tert*-Butoxycarbonyl)piperidin-4-yl)amino)-2-isopropoxypyrimidine-4-carboxylic acid (200 mg, 0.526 mmol, 1.0 equiv.) was dissolved in *N,N*-dimethylformamide (2 mL). (3*R*,4*R*)-4-(3,4-Dihydroisoquinolin-2(1*H*)-yl)piperidin-3-ol (146.6 mg, 0.631 mmol, 1.2 equiv.), HATU (299.8 mg, 0.789 mmol, 1.5 equiv.), and *N,* N-diisopropylethylamine (203.8 mg, 1.577 mmol, 3.0 equiv.) were added. The mixture was stirred at room temperature for 2 h. After the reaction was completed, as detected by LC-MS, extraction and concentration were performed, and the residue was separated by reverse-phase chromatography to give the compound (80 mg, yield: 25.6%).
LC-MS (ESI) $[M+H]^+$ = 595.4.

Step 4: Preparation of (3R,4R)-4-(3,4-dihydroisoquinolin-2(1H)-yl)-3-hydroxypiperidin-1-yl)(2-isopropoxy-6-(piperidin-4-ylamino)pyrimidin-4-yl)methanone

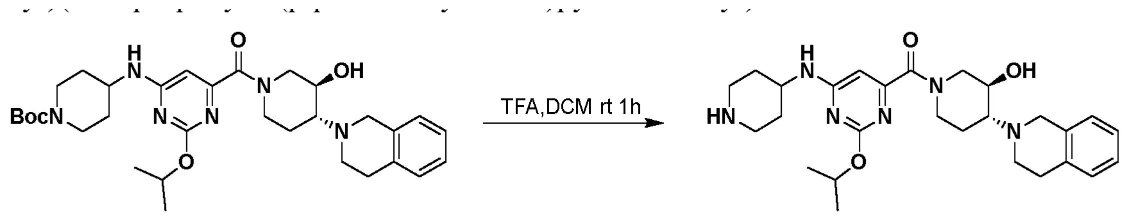


*tert-Butyl* 4-((6-((3*R*,4*R*)-4-(3,4-dihydroisoquinolin-2(1*H*)-yl)-3-hydroxypiperidine-1-carbonyl)-2-isopropoxypyrimidin-4-yl)amino)piperidine-1-carboxylate (80 mg, 0.134 mmol, 1.0 equiv.) was dissolved in dichloromethane (2 mL). Trifluoroacetic acid (0.5 mL) was added, and the mixture was stirred at 25 °C for 1 h. After the reaction was completed, as detected by LC-MS, the mixture was concentrated to give the title compound (70 mg, crude product).
LC-MS (ESI) $[M+H]^+$ = 495.3.

Step 5: Preparation of (6-((1-benzoylpiperidin-4-yl)amino)-2-isopropoxypyrimidin-4-yl)((3*R*,4*R*)-4-(3,4-dihydroisoquinolin-2(1*H*)-yl)-3-hydroxypiperidin-1-yl)methanone

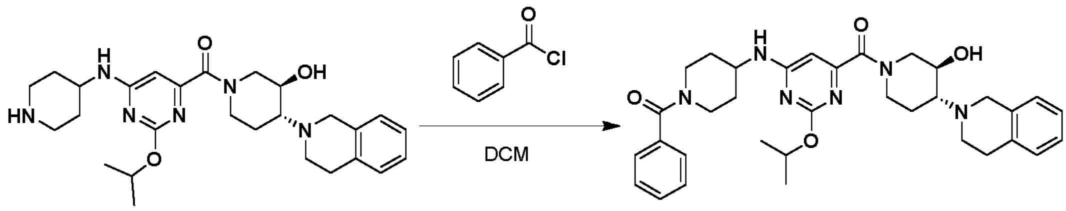


((3*R*,4*R*)-4-(3,4-Dihydroisoquinolin-2(1*H*)-yl)-3-hydroxypiperidin-1-yl)(2-isopropoxy-6-(piperidin-4-ylamino) pyrimidin-4-yl)methanone (70 mg, 0.142 mmol, 1.0 equiv.) was dissolved in dichloromethane (1 mL). *N,* N-Diisopropylethylamine (54.77 mg, 0.425 mmol, 3.0 equiv.) was added, and the mixture was stirred in an ice bath for 15 min. Benzoyl chloride (21.96 mg, 0.156 mmol, 1.1 equiv.) was then added, and the mixture was gradually warmed to room temperature and stirred for 2 h. After the reaction was completed, as detected by LC-MS, the mixture was concentrated to give a crude product, which was further separated by reverse-phase chromatography to give compound I (37 mg, yield: 43.7%).
The analysis was conducted using the aforementioned instruments 10 and 11, yielding the following results: LC-MS (ESI) $[M+H]^+$ = 599.3; $^1H$ NMR (400 MHz, $CDCl_3$) δ 7.49-7.36 (m, 5H), 7.14 (m, 3H), 7.07-6.95 (m, 1H), 6.21 (d, J = 22.4 Hz, 1H), 5.32-5.12 (m, 1H), 5.09-4.55 (m, 3H), 4.29 (m, 1H), 3.97 (m, 1H), 3.85-3.59 (m, 3H), 3.27-2.87 (m, 6H), 2.85-2.53 (m, 3H), 2.07-2.01 (m, 3H), 1.77-1.51 (m, 3H), 1.41-1.28 (m, 6H).
The analysis was conducted using the aforementioned instrument 4, and the XRPD pattern of compound I is shown in FIG. 17.

**Example 2:** Preparation of Salts of Compound I

Compound I prepared in Example 1 was used as a sample. 29.9 mg of compound I and 2 equivalents of 15 acidic compounds were separately weighed and added to a certain amount of a selected solvent. The mixture was subjected to suspension for 3 days at room temperature. The resulting suspension was separated by centrifugation, and the solid was dried under vacuum at room temperature. The resulting solids were analyzed by XRPD using the aforementioned instrument 5, and the results are shown in the table below.

| Solvent / Acidic compound | Methyl *tert*-butyl ether | Tetrahydrofuran/ cyclohexane (v/v, 1:1) | Ethanol/*n*-heptane (v/v, 3:7) | Methyl *tert*-butyl ether/acetone (v/v, 1:1) |
|---|---|---|---|---|
| Hydrobromic acid | Amorphous form | No solid | No solid | Gum |
| Sulfuric acid | Amorphous form | Oil | Oil | Gum |
| Phosphoric acid | Amorphous form | Gum | Gum | Amorphous form |
| Methanesulfonic acid | Amorphous form | Oil | No solid | Gum |
| *p*-Toluenesulfonic acid | Crystal form A of *p*-toluenesulfonate | Crystal form B of *p*-toluenesulfonate | Crystal form B of *p*-toluenesulfonate | Crystal form B of *p*-toluenesulfonate |
| *L*-Camphorsulfonic acid | Amorphous form | Gum | No solid | No solid |
| Oxalic acid | Amorphous form | Amorphous form | Crystal form A of oxalate | Crystal form A of oxalate |
| Maleic acid | Amorphous form | Gum | No solid | Gum |
| *L*-Tartaric acid | Tartrate + multimodal | Crystal form A of tartrate | Crystal form A of tartrate | Crystal form A of tartrate |
| Fumaric acid | Fumaric acid | Gum | No solid | Gum |
| Citric acid | Amorphous form | Gum | Gum | Amorphous form |
| *L*-Malic acid | Amorphous form | Gum | Oil | Gum |
| Glycolic acid | Amorphous form | Gum | No solid | No solid |
| Benzoic acid | Amorphous form | No solid | No solid | No solid |
| Hydrochloric acid | Crystal form A of hydrochloride | Crystal form A of hydrochloride | Crystal form A of hydrochloride + multimodal | Crystal form A of hydrochloride + multimodal |

As can be seen from the table, the following crystal forms of salts were found after screening: crystal form A of hydrochloride, crystal form A of *p*-toluenesulfonate, crystal form B of *p*-toluenesulfonate, crystal form A of tartrate, and crystal form A of oxalate.

**Example 3:** Preparation of Crystal Form A of Compound I

Compound I prepared in Example 1 was used as a sample. 19.8 mg of compound I was weighed and suspended in a mixed solvent of 0.2 mL of ethyl acetate and 0.8 mL of cyclohexane at room temperature for 7 days. The resulting suspension was separated by centrifugation, followed by drying to give a solid, which was designated as crystal form A of compound I.

The XRPD pattern of the crystal form A of compound I, determined using the aforementioned instrument 4, is shown in FIG. 1, with the corresponding diffraction peaks data listed below. The DSC and TGA profiles, determined using the aforementioned instruments 1 and 2, are shown in FIG. 2.

| #Peak | 2θ angle (°) | Intensity (%) | #Peak | 2θ angle (°) | Intensity (%) |
|---|---|---|---|---|---|
| 1 | 4.01 | 98.5 | 6 | 16.22 | 64.9 |
| 2 | 7.10 | 50.5 | 7 | 18.37 | 80.0 |
| 3 | 13.63 | 58.5 | 8 | 20.28 | 100.0 |
| 4 | 14.68 | 64.6 | 9 | 21.83 | 80.9 |
| 5 | 15.79 | 66.2 | 10 | 27.68 | 32.1 |

**Example 4:** Crystal Form A of Hydrochloride of Compound I

Compound I prepared in Example 1 was used as a sample. 28.5 mg (about 0.05 mmol) of compound I and 2 equivalents of hydrochloric acid (calculated based on HCl) were weighed and added to 1 mL of a mixed solvent of tetrahydrofuran/cyclohexane (v/v, 1:1). The mixture was stirred at room temperature for 2 days. The resulting suspension was separated by centrifugation, and the solid was dried to give a new crystal form of the hydrochloride of compound I, which was designated as crystal form A of the hydrochloride of compound I.

The ion chromatography results determined using the aforementioned instrument 8 show that the chloride ion content was 10.2%, which was consistent with the theoretical content of 10.4% for 2 equivalents of chloride ions. Therefore, the salt-forming ratio of compound I to hydrochloric acid (calculated based on HCl) was 1:2.

The XRPD pattern of the crystal form A of the hydrochloride of compound I, determined using the aforementioned instrument 5, is shown in FIG. 3, with the corresponding diffraction peaks data listed below. The DSC and TGA profiles, determined using the aforementioned instruments 1 and 2, are shown in FIG. 4.

| #Peak | 2θ angle (°) | Intensity (%) | #Peak | 2θ angle (°) | Intensity (%) |
|---|---|---|---|---|---|
| 1 | 4.42 | 71.2 | 26 | 26.58 | 29.9 |
| 2 | 7.20 | 26.9 | 27 | 26.73 | 37.4 |
| 3 | 8.49 | 39.2 | 28 | 26.96 | 28.0 |
| 4 | 8.86 | 23.9 | 29 | 27.66 | 12.4 |
| 5 | 11.47 | 44.0 | 30 | 28.22 | 18.1 |
| 6 | 12.74 | 43.2 | 31 | 28.66 | 13.1 |
| 7 | 13.95 | 39.7 | 32 | 29.56 | 22.8 |
| 8 | 14.45 | 26.1 | 33 | 30.10 | 8.7 |
| 9 | 14.65 | 35.8 | 34 | 30.95 | 12.6 |
| 10 | 15.29 | 61.2 | 35 | 31.68 | 11.9 |
| 11 | 16.66 | 36.7 | 36 | 32.61 | 16.5 |
| 12 | 17.02 | 16.3 | 37 | 33.03 | 21.1 |
| 13 | 18.02 | 100.0 | 38 | 33.84 | 9.2 |
| 14 | 18.81 | 66.6 | 39 | 34.88 | 16.6 |
| 15 | 19.33 | 80.5 | 40 | 35.41 | 7.7 |
| 16 | 19.66 | 22.8 | 41 | 36.44 | 9.1 |
| 17 | 20.91 | 35.5 | 42 | 37.20 | 14.3 |
| 18 | 21.62 | 43.2 | 43 | 38.39 | 12.8 |
| 19 | 22.28 | 19.2 | 44 | 39.15 | 8.0 |
| 20 | 22.85 | 25.3 | 45 | 40.22 | 6.5 |
| 21 | 23.25 | 37.9 | 46 | 41.09 | 13.7 |
| 22 | 23.56 | 18.5 | 47 | 42.63 | 6.1 |
| 23 | 24.47 | 8.9 | 48 | 43.60 | 6.0 |

(continued)

| #Peak | 2θ angle (°) | Intensity (%) | #Peak | 2θ angle (°) | Intensity (%) |
|---|---|---|---|---|---|
| 24 | 25.56 | 28.2 | 49 | 44.54 | 7.5 |
| 25 | 26.20 | 25.9 | | | |

**Example 5:** Crystal Form A of Oxalate of Compound I

Compound I prepared in Example 1 was used as a sample. 29.6 mg (about 0.05 mmol) of compound I and 2 equivalents of oxalic acid were weighed and added to 1 mL of a mixed solvent of ethanol/n-heptane (v/v, 3:7). The mixture was stirred at room temperature for 3 days. The resulting suspension was separated by centrifugation, and the solid was dried to give a new crystal form of the oxalate of compound I, which was designated as crystal form A of the oxalate of compound I.

The XRPD pattern of the crystal form A of the oxalate of compound I, determined using the aforementioned instrument 5, is shown in FIG. 5, with the corresponding diffraction peaks data listed below. The DSC and TGA profiles, determined using the aforementioned instruments 1 and 2, are shown in FIG. 6.

| #Peak | 2θ angle (°) | Intensity (%) | #Peak | 2θ angle (°) | Intensity (%) |
|---|---|---|---|---|---|
| 1 | 4.51 | 92.4 | 15 | 20.14 | 95.4 |
| 2 | 5.15 | 59.9 | 16 | 21.20 | 91.6 |
| 3 | 6.07 | 55.4 | 17 | 22.29 | 61.7 |
| 4 | 8.37 | 100.0 | 18 | 23.01 | 60.8 |
| 5 | 10.39 | 59.6 | 19 | 24.31 | 44.2 |
| 6 | 12.22 | 49.8 | 20 | 25.70 | 44.2 |
| 7 | 12.73 | 44.4 | 21 | 27.33 | 29.2 |
| 8 | 14.87 | 40.9 | 22 | 28.80 | 26.7 |
| 9 | 15.74 | 54.6 | 23 | 29.90 | 24.1 |
| 10 | 16.27 | 59.9 | 24 | 31.86 | 20.3 |
| 11 | 16.75 | 75.9 | 25 | 34.20 | 19.8 |
| 12 | 17.02 | 69.0 | 26 | 35.80 | 16.4 |
| 13 | 17.61 | 65.8 | 27 | 38.20 | 16.1 |
| 14 | 18.77 | 93.9 | | | |

**Example 6:** Crystal Form A of Tartrate of Compound I

Compound I prepared in Example 1 was used as a sample. 29.9 mg (about 0.05 mmol) of compound I and 2 equivalents of tartaric acid were weighed and added to 1 mL of a mixed solvent of tetrahydrofuran/cyclohexane (v/v, 1:1). The mixture was subjected to suspension at room temperature for 3 days. The resulting suspension was separated by centrifugation, and the solid was dried to give a new crystal form of the tartrate of compound I, which was designated as crystal form A of the tartrate of compound I.

The XRPD pattern of the crystal form A of the tartrate of compound I, determined using the aforementioned instrument 5, is shown in FIG. 7, with the corresponding diffraction peaks data listed below. The DSC and TGA profiles, determined using the aforementioned instruments 1 and 2, are shown in FIG. 8. The NMR spectrum, determined using the aforementioned instrument 7, is shown in FIG. 9, with a tartaric acid signal peak observed at 4.25 ppm. Based on the integration results, the salt-forming ratio of compound I to tartaric acid was determined to be 1:1.5.

| #Peak | 2θ angle (°) | Intensity (%) | #Peak | 2θ angle (°) | Intensity (%) |
|---|---|---|---|---|---|
| 1 | 3.04 | 74.3 | 17 | 20.53 | 84.6 |
| 2 | 5.11 | 39.3 | 18 | 21.19 | 59.2 |

(continued)

| #Peak | 2θ angle (°) | Intensity (%) | #Peak | 2θ angle (°) | Intensity (%) |
|---|---|---|---|---|---|
| 3 | 5.77 | 53.7 | 19 | 22.42 | 48.0 |
| 4 | 6.68 | 37.1 | 20 | 22.81 | 44.3 |
| 5 | 8.82 | 56.4 | 21 | 23.81 | 49.5 |
| 6 | 10.16 | 35.1 | 22 | 24.34 | 43.3 |
| 7 | 10.77 | 40.0 | 23 | 25.35 | 35.3 |
| 8 | 11.73 | 51.1 | 24 | 25.74 | 35.3 |
| 9 | 13.19 | 45.5 | 25 | 26.75 | 28.0 |
| 10 | 14.65 | 41.9 | 26 | 27.86 | 23.2 |
| 11 | 15.55 | 94.3 | 27 | 28.87 | 25.7 |
| 12 | 15.95 | 100.0 | 28 | 31.37 | 20.8 |
| 13 | 16.73 | 57.8 | 29 | 32.74 | 18.9 |
| 14 | 17.38 | 49.8 | 30 | 33.91 | 18.3 |
| 15 | 18.35 | 99.3 | 31 | 35.14 | 17.2 |
| 16 | 18.72 | 78.7 | 32 | 37.55 | 19.0 |

**Example 7:** Crystal Form A of *p*-toluenesulfonate of Compound I

Compound I prepared in Example 1 was used as a sample. 29.8 mg (about 0.05 mmol) of compound I and 2 equivalents of *p*-toluenesulfonic acid were weighed and added to 1 mL of methyl *tert*-butyl ether. The mixture was stirred at room temperature for 3 days. The resulting suspension was separated by centrifugation, and the solid was dried to give a new crystal form of the *p*-toluenesulfonate of compound I, which was designated as crystal form A of the *p*-toluenesulfonate of compound I.

The XRPD pattern of the crystal form A of the *p*-toluenesulfonate of compound I, determined using the aforementioned instrument 5, is shown in FIG. 10, with the corresponding diffraction peaks data listed below. The DSC and TGA profiles, determined using the aforementioned instruments 1 and 2, are shown in FIG. 11. The NMR spectrum, determined using the aforementioned instrument 7, is shown in FIG. 12, with *p*-toluenesulfonic acid signal peaks observed at 2.27 ppm, 7.10 ppm, and 7.45 ppm. Based on the integration results, the salt-forming ratio of compound I to*p*-toluenesulfonic acid was determined to be 1:2.

| #Peak | 2θ angle (°) | Intensity (%) | #Peak | 2θ angle (°) | Intensity (%) |
|---|---|---|---|---|---|
| 1 | 3.97 | 100.0 | 11 | 18.34 | 52.3 |
| 2 | 6.19 | 24.5 | 12 | 19.01 | 49.0 |
| 3 | 8.07 | 43.5 | 13 | 20.03 | 65.0 |
| 4 | 8.83 | 36.0 | 14 | 20.44 | 51.4 |
| 5 | 11.65 | 24.4 | 15 | 23.10 | 29.9 |
| 6 | 12.72 | 27.6 | 16 | 24.44 | 22.3 |
| 7 | 14.55 | 27.2 | 17 | 25.90 | 19.6 |
| 8 | 15.30 | 23.8 | 18 | 27.33 | 14.7 |
| 9 | 16.34 | 26.6 | 19 | 29.16 | 15.1 |
| 10 | 17.51 | 34.3 | 20 | 30.85 | 12.1 |

**Example 8:** Crystal Form B of *p*-toluenesulfonate of Compound I

Compound I prepared in Example 1 was used as a sample. 29.9 mg (about 0.05 mmol) of compound I and 2

equivalents ofp-toluenesulfonic acid were weighed and added to 1 mL of a mixed solvent of tetrahydrofuran/cyclohexane (v/v, 1:1). The mixture was stirred at room temperature for 3 days. The resulting suspension was separated by centrifugation, and the solid was dried to give a new crystal form of the *p*-toluenesulfonate of compound I, which was designated as crystal form B of the p-toluenesulfonate of compound I. The XRPD pattern of the crystal form B of the p-toluenesulfonate of compound I, determined using the aforementioned instrument 5, is shown in FIG. 13, with the corresponding diffraction peaks data listed below. The NMR spectrum, determined using the aforementioned instrument 7, is shown in FIG. 14, with *p*-toluenesulfonic acid signal peaks observed at 2.27 ppm, 7.10 ppm, and 7.45 ppm. Based on the integration results, the salt-forming ratio of compound I to *p*-toluenesulfonic acid was determined to be 1:2.

| #Peak | 2θ angle (°) | Intensity (%) | #Peak | 2θ angle (°) | Intensity (%) |
|---|---|---|---|---|---|
| 1 | 4.32 | 67.8 | 20 | 20.24 | 89.8 |
| 2 | 4.90 | 24.4 | 21 | 21.04 | 14.5 |
| 3 | 5.78 | 3.1 | 22 | 21.53 | 36.4 |
| 4 | 7.14 | 12.6 | 23 | 22.44 | 9.5 |
| 5 | 8.59 | 21.1 | 24 | 23.00 | 11.6 |
| 6 | 8.99 | 21.8 | 25 | 23.67 | 12.1 |
| 7 | 9.79 | 56.5 | 26 | 24.24 | 10.7 |
| 8 | 10.72 | 5.5 | 27 | 24.75 | 12.8 |
| 9 | 11.50 | 40.5 | 28 | 25.33 | 11.5 |
| 10 | 12.81 | 14.9 | 29 | 25.74 | 23.4 |
| 11 | 14.32 | 5.9 | 30 | 27.43 | 5.5 |
| 12 | 14.84 | 11.3 | 31 | 28.51 | 4.5 |
| 13 | 15.37 | 2.8 | 32 | 29.14 | 12.5 |
| 14 | 16.82 | 24.6 | 33 | 29.52 | 13.2 |
| 15 | 17.20 | 32.2 | 34 | 30.90 | 6.1 |
| 16 | 17.77 | 9.4 | 35 | 32.10 | 5.3 |
| 17 | 18.21 | 12.1 | 36 | 35.58 | 2.9 |
| 18 | 19.12 | 100.0 | 37 | 37.78 | 2.9 |
| 19 | 19.56 | 18.6 | 38 | 38.79 | 1.7 |

**Example 9:** Crystal Form B ofp-toluenesulfonate of Compound I

Method 1:

Compound I prepared in Example 1 was used as a sample. 2.9 g (about 5 mmol) of compound I and 2.5 equivalents of *p*-toluenesulfonic acid were weighed and added to 45 mL of acetone. The mixture was stirred at room temperature for 2 h. The resulting suspension was separated by centrifugation, and the solid was dried to give a *p*-toluenesulfonate crystal form of compound I. The XRPD pattern, determined using the aforementioned instrument 6, is shown in FIG. 15, confirming that the obtained crystal form corresponded to crystal form B of the *p*-toluenesulfonate of compound I. The DSC and TGA profiles, determined using the aforementioned instrument 3, are shown in FIG. 16. Based on the TGA weight loss results, it was determined that the crystal form B contained one molecule of water of crystallization.

Method 2:

Compound I prepared in Example 1 was used as a sample. 598.1 mg of compound I and 343.9 mg of *p*-toluenesulfonic acid were weighed and added to 20 mL of a mixed solvent of methyl tert-butyl ether/acetone (v/v, 1:1). The mixture was subjected to suspension at room temperature for 2 days. The resulting suspension was separated by filtration under vacuum, and the solid was dried under vacuum at 40 °C to give a crystal form, which was identical to the crystal form B of the p-toluenesulfonate of compound I described above. The above results indicate that for the crystal form B of the p-

toluenesulfonate of compound I, the MTBE/acetone binary solvent system used in the salt formation reaction can be further simplified to a single acetone solvent system.

**Test Example 1:** Evaluation of Inhibitory Activity Against PRMT5 Enzymatic Activity

1. Test method:

A 1× enzyme reaction buffer was prepared using 10 mM Tris 8.0 (Sigma, Cat. No. T2694-1L), 0.01% Tween-20 (Sigma, Cat. No. P2287-100ML), and 1 mM DTT (Sigma, Cat. No. D0632-10G). PRMT5 (Active Motif, Cat. No. 31921) and [3H]-SAM (PerkinElmer, Cat. No. NET155V001MC) were added to the 1× enzyme reaction buffer to prepare a 25/15× mixed solution (final PRMT5 concentration: 5 nM; final [3H]-SAM concentration: 0.3 μM). 15 μL of this solution was transferred into a 384-well microplate (Corning 384-well Polypropylene Storage Microplates, Cat. No. 3657) containing different concentrations of the compound (final DMSO concentration: 1%) and incubated at room temperature for 60 min. The polypeptide substrate GL-27 (Ac-SGRGKGGKGLGKGGAKRHRKVGG-K)(Biotin) (GL Biochem, Cat. No. 342095) was added to the 1× enzyme reaction buffer to prepare a 25/10× substrate solution. Then, 10 μL of the polypeptide substrate solution was added to the reaction system (final polypeptide substrate concentration: 100 nM), and the mixture was incubated at room temperature for 120 min. The reaction was terminated by adding 5 μL of a 6× cold SAM solution (Sigma, Cat. No. A7007-100MG) (final SAM concentration: 0.125 mM). A 25 μL aliquot of the reaction system was transferred to a FlashPlate (Streptavidin FlashPlate HTS PLUS, High Capacity, 384-well, PerkinElmer, Cat. No. SMP410A001PK) and incubated at room temperature for 1 h. Then, the FlashPlate was washed three times with distilled water containing 0.1% Tween-20, and the CPM (Counts Per Minute) data was recorded using a MicroBeta instrument. After obtaining the raw CPM data for different concentrations of the compound, the data were normalized using the following formula to calculate the enzymatic activity inhibition rate (Inh%) at each concentration: Inh% = (Max - Sample)/(Max - Min) * 100% (wherein Max represents the CPM value of the positive control wells containing the enzyme, Min represents the CPM value of the negative control wells without the enzyme, and Sample represents the CPM value of the sample wells treated with the compound). Subsequently, the concentration (X) and corresponding inhibition rate (Inh%) (Y) were input into EXCEL. The XLFit plug-in was then used to fit the data using the built-in four-parameter logistic equation: Y = Bottom + (Top - Bottom)/(1 + ($IC_{50}$/X) * HillSlope). The $IC_{50}$ (half-maximal inhibitory concentration) value for each compound was then calculated.

2. Test results:

The compound from Example 1 of the present disclosure exhibited an $IC_{50}$ value of 6.40 nM in the PRMT5 enzymatic activity inhibition test, demonstrating excellent biological activity. Compound 13 disclosed in WO2020182018A1 was used as a reference compound (the structure and preparation method of the reference compound can be found in Example 13 on pages 33-35 of the specification of WO2020182018A1). Using the same test method, the $IC_{50}$ value of the reference compound was determined to be 115.00 nM.

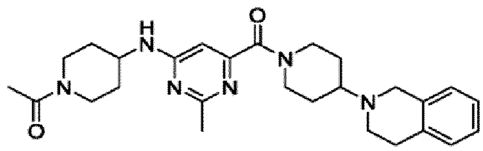

13

**Test Example 2:** Evaluation of Growth Inhibitory Activity in Human Origin B-Cell Non-Hodgkin's Lymphoma Z-138 Cells

1. Experimental materials and instruments

1) Cell line and culture method

| Cell line | Supplier | Cat. No. | Tumor type | Characteristics of growth | Culture method |
|---|---|---|---|---|---|
| Z-138 | ATCC | CRL-3001 | Mantle cell lymphoma (B-cell non-Hodgkin's lymphoma) | Suspension | IMDM + 10% FBS |

2) Culture medium and reagents

| Culture medium and reagent | Manufacturer | Cat. No. |
|---|---|---|
| IMDM | GIBCO | 31980048 |
| FBS | Hyclone | SH30084.03 |
| Penicillin-streptomycin | Thermo | SV30010 |
| DMSO | SIGMA | D2650 |
| Promega CellTiter-Glo luminescent cell viability assay kit | Promega | Promega-G7573 |

3) 384-well plate

Corning® 384-well clear flat bottom white polystyrene microplate (with lid, sterile), Corning, Cat. No.: 3765.

4) Instruments

2104 EnVision plate reader, PerkinElmer;
Vi-Cell XR cell counter, Beckman Coulter.

2. Experimental method and procedures

1) Cell culturing

The cells were thawed and cultured in an incubator at 37 °C with 5% $CO_2$ under the conditions listed in the table above. The cells were routinely subcultured. After approximately two passages, well-growing cell strains were selected for plating.

2) Cell plating

I. The suspension was removed from the incubator, transferred into a 50 mL centrifuge tube, and then centrifuged at 800-1000 rpm for 3-5 min, and the supernatant was discarded. An appropriate volume of culture medium was added to the centrifuge tube, and the cells were gently pipetted to ensure a uniform suspension. Cell counting was performed using the Vi-Cell XR cell counter.
II. Based on the measured cell density, the cell suspension was adjusted to an appropriate concentration.
III. The cell suspension was added to a 384-well plate at a volume of 40 μL/well, with 700 cells/well. For blank control wells, an equal volume of cell-free culture medium was added instead.

3) Compound preparation and dosing

I. The compound was dissolved in 100% DMSO to prepare a 10 mM stock solution.
II. The 10 mM stock solution was diluted with DMSO to a concentration of 2 mM, which was then used as the starting concentration for 4× serial dilutions across 9 points using DMSO.

III. 200 nL of each gradient concentration solution of the above compound was added to each well. For blank control and DMSO control wells, 200 nL of DMSO was added instead, ensuring a final DMSO concentration of 0.5%.
IV. The cell plate was incubated in a $CO_2$ incubator for 5 days (120 h).

4) Reagent preparation and assay

The assay was performed according to the instructions of the Promega CellTiter-Glo luminescent cell viability assay kit (Promega-G7573):

I. The CellTiter-Glo buffer was thawed and let stand at room temperature.
II. The lyophilized CellTiter-Glo substrate was let stand at room temperature.
III. The CellTiter-Glo buffer was added to a flask of CellTiter-Glo substrate to dissolve the substrate, thereby preparing a CellTiter-Glo working solution.
IV. The solution was slowly vortexed to ensure complete dissolution.
V. The cell culture plate was removed and allowed to equilibrate to room temperature.
VI. 25 μL of the mixed CellTiter-Glo reagent was added to each well, and the plate was shaken in the dark for 10 min, followed by incubation for 10 min.
VII. The luminescence signals were detected on 2104 EnVision plate reader.

3. Data analysis

The inhibition rate (IR) of the compound was calculated using the following formula:

$$IR\ (\%) = (1 - (RLU_{compound} - RLU_{blank\ control})/(RLU_{DMSO} - RLU_{blank\ control})) * 100\%.$$

The dose-response inhibition curve was plotted using XLFit, and $IC_{50}$ values were calculated using the four-parameter logistic model: [fit = (A + ((B - A)/(1 + ((C/x) ^ D))))].

4. Experimental results:

The compound from Example 1 of the present disclosure exhibited an $IC_{50}$ value of 0.0466 μM in the Z-138 cell growth inhibition test, demonstrating excellent biological activity. Similarly, compound 13 disclosed in WO2020182018A1 was used as a reference compound. Using the same test method, the $IC_{50}$ value of the reference compound was determined to be 2.7096 μM.

**Test Example 3:** Stability Study

A stability study was conducted on the crystal form B of the p-toluenesulfonate, the crystal form A of the tartrate, and the crystal form A of the hydrochloride disclosed herein under high temperature (60 °C), high humidity (25 °C/92.5% RH), light exposure (25 °C/4500 Lux), and accelerated conditions (40 °C/75% RH), with samples collected at day 7 and day 15 for analysis. The results are shown below.

| Salt form | Condition | Day 7 result | Day 15 result | Purity (%) | | |
|---|---|---|---|---|---|---|
| | | | | day 0 | day 7 | day 15 |
| Crystal form B of *p*-toluenesulfonate | 60 °C | Unchanged | Unchanged | 99.15 | 98.57 | 97.18 |
| | 25 °C/92.5% RH | Unchanged | Unchanged | | 98.85 | 97.54 |
| | 25 °C/4500 Lux | Unchanged | Unchanged | | 98.69 | 97.72 |
| | 40 °C/75% RH | Unchanged | Unchanged | | 99.02 | 98.52 |
| Crystal form A of tartrate | 60 °C | Unchanged | Unchanged | 99.01 | 98.68 | 98.14 |
| | 25 °C/92.5% RH | Unchanged | Unchanged | | 98.94 | 98.70 |
| | 25 °C/4500 Lux | Unchanged | Unchanged | | 98.70 | 98.27 |
| | 40 °C/75% RH | Unchanged | Unchanged | | 98.96 | 98.86 |

(continued)

| Salt form | Condition | Day 7 result | Day 15 result | Purity (%) | | |
|---|---|---|---|---|---|---|
| | | | | day 0 | day 7 | day 15 |
| Crystal form A of hydrochloride | 60 °C | Unchanged | Unchanged | 98.44 | 95.96 | 94.87 |
| | 25 °C/92.5% RH | Polymorphic transition | Polymorphic transition | | 98.72 | 98.55 |
| | 25 °C/4500 Lux | Unchanged | Unchanged | | 98.43 | 97.90 |
| | 40 °C/75% RH | Unchanged | Unchanged | | 98.57 | 98.48 |

The results show that the crystal form B of the p-toluenesulfonate and the crystal form A of the tartrate remained stable without polymorphic transition under high temperature, high humidity, light exposure, and accelerated conditions for 15 days, whereas the crystal form A of the hydrochloride exhibited polymorphic transition after 7 and 15 days under high humidity conditions, while remaining stable under all other conditions.

**Test Example 4:** Hygroscopicity Study

The crystal form B of the p-toluenesulfonate, the crystal form A of the tartrate, and the crystal form A of the hydrochloride disclosed herein were subjected to dynamic vapor sorption/desorption analysis using the aforementioned instrument 9 to evaluate their hygroscopicity. The results are shown below.

| Salt form | Condition | Test result |
|---|---|---|
| Crystal form B of *p*-toluenesulfonate | 80% humidity | Weight gain 0.17% |
| | 95% humidity | Weight gain 0.54% |
| Crystal form A of tartrate | 80% humidity | Weight gain 2.85% |
| | 95% humidity | Weight gain 12.95% |
| Crystal form A of hydrochloride | 80% humidity | Weight gain 2.46% |
| | 95% humidity | Weight gain 7.57% |

The results show that, in terms of hygroscopicity, the ranking from lowest to highest was as follows: the crystal form B of the *p*-toluenesulfonate < the crystal form A of the hydrochloride < the crystal form A of the tartrate. Among them, the crystal form B of the *p*-toluenesulfonate was found to be almost non-hygroscopic.

The embodiments of the present disclosure have been described above. However, the above embodiments are not intended to limit the present disclosure. Any modification, equivalent replacement, improvement, and the like made without departing from the spirit and principle of the present disclosure shall fall within the protection scope of the present disclosure.

## Claims

**1.** A crystal form A of compound I, wherein the structure of compound I is as follows:

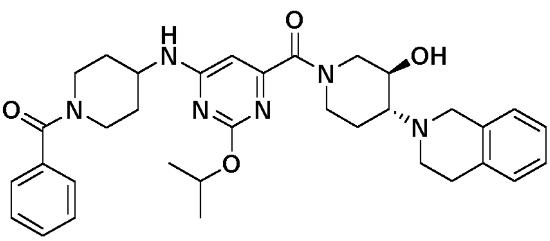

;

the crystal form A has characteristic peaks at 2θ angles of 4.0±0.2°, 18.4±0.2°, 20.3±0.2°, and 21.8±0.2° in an XRPD pattern using Cu-Kα radiation;

preferably, the crystal form A has characteristic peaks at 2θ angles of 4.0±0.2°, 14.7±0.2°, 15.8±0.2°, 18.4 ±0.2°, 20.3±0.2°, and 21.8±0.2° in the XRPD pattern using Cu-Kα radiation;
more preferably, the crystal form A has characteristic peaks at 2θ angles of 4.0±0.2°, 7.1±0.2°, 13.6±0.2°, 14.7 ±0.2°, 15.8±0.2°, 18.4±0.2°, 20.3±0.2°, 21.8±0.2°, and 27.7±0.2° in the XRPD pattern using Cu-Kα radiation;
further preferably, the crystal form A has an XRPD pattern substantially as shown in FIG. 1.

**2.** The crystal form A of compound I as claimed in claim 1, wherein

a DSC profile of the crystal form A has an endothermic peak at around 85.0 °C to 150.0 °C;
preferably, the DSC profile of the crystal form A has an endothermic peak at 85.0 °C ± 5 °C to 150.0 °C ± 5 °C;
more preferably, the crystal form A has a DSC profile substantially as shown in FIG. 2.

**3.** The crystal form A of compound I as claimed in claim 1 or 2, wherein

a TGA profile of the crystal form A shows a weight loss of about 2.0% at around 100.0 °C, and also shows a weight loss of about 3.5% at around 150.0 °C;
preferably, the TGA profile of the crystal form A shows a weight loss of about 2.0% at 100.0 °C ± 5 °C, and also shows a weight loss of about 3.5% at 150.0 °C ± 5 °C;
more preferably, the crystal form A has a TGA profile substantially as shown in FIG. 2.

**4.** A salt of compound I, wherein the structure of compound I is as follows:

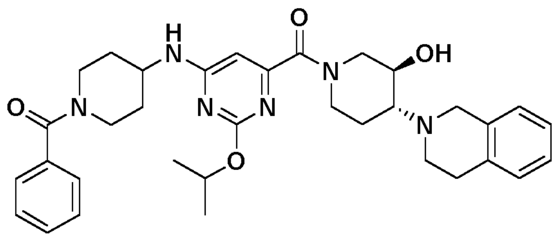

;

the salt is a hydrochloride, hydrobromide, phosphate, sulfate, methanesulfonate, p-toluenesulfonate, camphorsulfonate (e.g., L-camphorsulfonate), oxalate, maleate, tartrate (e.g., L-tartrate), fumarate, citrate, malate (e.g., L-malate), glycolate or benzoate of compound I.

**5.** A hydrochloride of compound I, wherein the structure of compound I is as follows:

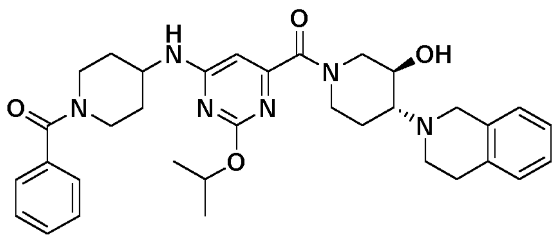

;

in the hydrochloride, the salt-forming ratio of compound I to hydrochloric acid is 1:2.

**6.** A crystal form A of a hydrochloride of compound I, wherein the structure of compound I is as follows:

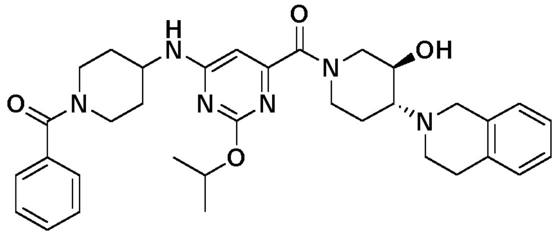

;

in the crystal form A of the hydrochloride, the salt-forming ratio of compound I to hydrochloric acid is 1:2.

**7.** The crystal form A of the hydrochloride of compound I as claimed in claim 6, wherein the crystal form A of the hydrochloride of compound I has characteristic peaks at 2θ angles of 4.4±0.2°, 15.3±0.2°, 18.0±0.2°, 18.8±0.2°, and 19.3±0.2° in an XRPD pattern using Cu-Kα radiation;

preferably, the crystal form A of the hydrochloride of compound I has characteristic peaks at 2θ angles of 4.4 ±0.2°, 11.5±0.2°, 12.7±0.2°, 15.3±0.2°, 18.0±0.2°, 18.8±0.2°, 19.3±0.2°, and 21.6±0.2° in the XRPD pattern using Cu-Kα radiation;
more preferably, the crystal form A of the hydrochloride of compound I has characteristic peaks at 2θ angles of 4.4 ±0.2°, 8.5±0.2°, 11.5±0.2°, 12.7±0.2°, 14.0±0.2°, 15.3±0.2°, 16.7±0.2°, 18.0±0.2°, 18.8±0.2°, 19.3±0.2°, 20.9±0.2°, 21.6±0.2°, and 23.3±0.2° in the XRPD pattern using Cu-Kα radiation;
further preferably, the crystal form A of the hydrochloride of compound I has an XRPD pattern substantially as shown in FIG. 3.

**8.** The crystal form A of the hydrochloride of compound I as claimed in claim 6 or 7, wherein

a DSC profile of the crystal form A of the hydrochloride of compound I has an endothermic peak at around 149.4 °C;
preferably, the DSC profile of the crystal form A of the hydrochloride of compound I has an endothermic peak at 149.4 °C ± 5 °C;
more preferably, the crystal form A of the hydrochloride of compound I has a DSC profile substantially as shown in FIG. 4.

**9.** The crystal form A of the hydrochloride of compound I as claimed in any one of claims 6 to 8, wherein

a TGA profile of the crystal form A of the hydrochloride of compound I shows a weight loss of about 0.3% at around 100.0 °C, and also shows a weight loss of about 16.3% at around 235.0 °C;
preferably, the TGA profile of the crystal form A of the hydrochloride of compound I shows a weight loss of about 0.3% at 100.0 °C ± 5 °C, and also shows a weight loss of about 16.3% at 235.0 °C ± 5 °C;
more preferably, the crystal form A of the hydrochloride of compound I has a TGA profile substantially as shown in FIG. 4.

**10.** A crystal form A of an oxalate of compound I, wherein the structure of compound I is as follows:

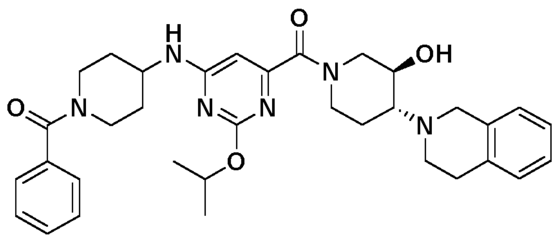

;

the crystal form A of the oxalate of compound I has characteristic peaks at 2θ angles of 4.5±0.2°, 8.4±0.2°, 18.8 ±0.2°, 20.1±0.2°, and 21.2±0.2° in an XRPD pattern using Cu-Kα radiation;
preferably, the crystal form A of the oxalate of compound I has characteristic peaks at 2θ angles of 4.5±0.2°, 6.1 ±0.2°, 8.4±0.2°, 10.4±0.2°, 16.8±0.2°, 18.8±0.2°, 20.1±0.2°, and 21.2±0.2° in the XRPD pattern using Cu-Kα

radiation;
more preferably, the crystal form A of the oxalate of compound I has characteristic peaks at 2θ angles of 4.5±0.2°, 5.2±0.2°, 6.1±0.2°, 8.4±0.2°, 10.4±0.2°, 15.8±0.2°, 16.3±0.2°, 16.8±0.2°, 17.6±0.2°, 18.8±0.2°, 20.1±0.2°, 21.2±0.2°, 22.3±0.2°, and 23.0±0.2° in the XRPD pattern using Cu-Kα radiation;
further preferably, the crystal form A of the oxalate of compound I has an XRPD pattern substantially as shown in FIG. 5.

**11.** The crystal form A of the oxalate of compound I as claimed in claim 10, wherein

a DSC profile of the crystal form A of the oxalate of compound I has an endothermic peak at around 157.2 °C;
preferably, the DSC profile of the crystal form A of the oxalate of compound I has an endothermic peak at 157.2 °C ± 5 °C;
more preferably, the crystal form A of the oxalate of compound I has a DSC profile substantially as shown in FIG. 6.

**12.** The crystal form A of the oxalate of compound I as claimed in claim 10 or 11, wherein a TGA profile of the crystal form A of the oxalate of compound I shows a weight loss of about 4.0% at around 150.0 °C;

preferably, the TGA profile of the crystal form A of the oxalate of compound I shows a weight loss of about 4.0% at 150.0 °C ± 5 °C;
more preferably, the crystal form A of the oxalate of compound I has a TGA profile substantially as shown in FIG. 6.

**13.** A tartrate of compound I, wherein the structure of compound I is as follows:

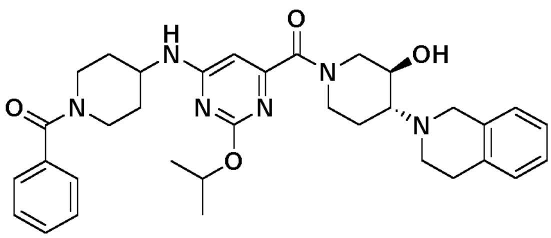
;

in the tartrate, the salt-forming ratio of compound I to tartaric acid is 1:1.5.

**14.** A crystal form A of a tartrate of compound I, wherein the structure of compound I is as follows:

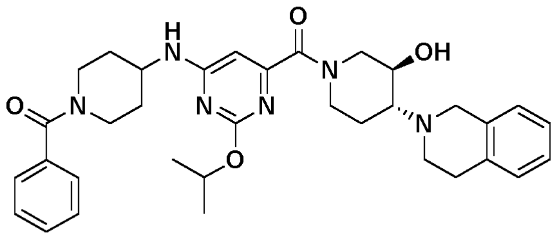
;

in the crystal form A of the tartrate, the salt-forming ratio of compound I to tartaric acid is 1:1.5.

**15.** The crystal form A of the tartrate of compound I as claimed in claim 14, wherein

the crystal form A of the tartrate of compound I has characteristic peaks at 2θ angles of 15.5±0.2°, 16.0±0.2°, 18.4±0.2°, and 20.5±0.2° in an XRPD pattern using Cu-Kα radiation;
preferably, the crystal form A of the tartrate of compound I has characteristic peaks at 2θ angles of 5.8±0.2°, 8.8 ±0.2°, 11.7±0.2°, 15.5±0.2°, 16.0±0.2°, 18.4±0.2°, and 20.5±0.2° in the XRPD pattern using Cu-Kα radiation;
more preferably, the crystal form A of the tartrate of compound I has characteristic peaks at 2θ angles of 3.0±0.2°, 5.8±0.2°, 8.8±0.2°, 11.7±0.2°, 15.5±0.2°, 16.0±0.2°, 16.7±0.2°, 18.4±0.2°, 20.5±0.2°, and 21.2±0.2° in the XRPD pattern using Cu-Kα radiation;
further preferably, the crystal form A of the tartrate of compound I has an XRPD pattern substantially as shown in

FIG. 7.

**16.** The crystal form A of the tartrate of compound I as claimed in claim 14 or 15, wherein a DSC profile of the crystal form A of the tartrate of compound I has an endothermic peak at around 174.8 °C;

preferably, the DSC profile of the crystal form A of the tartrate of compound I has an endothermic peak at 174.8 °C ± 5 °C;
more preferably, the crystal form A of the tartrate of compound I has a DSC profile substantially as shown in FIG. 8.

**17.** The crystal form A of the tartrate of compound I as claimed in any one of claims 14 to 16, wherein

a TGA profile of the crystal form A of the tartrate of compound I shows a weight loss of about 5.8% at around 150.0 °C;
preferably, the TGA profile of the crystal form A of the tartrate of compound I shows a weight loss of about 5.8% at 150.0 °C ± 5 °C;
more preferably, the crystal form A of the tartrate of compound I has a TGA profile substantially as shown in FIG. 8.

**18.** A p-toluenesulfonate of compound I, wherein the structure of compound I is as follows:

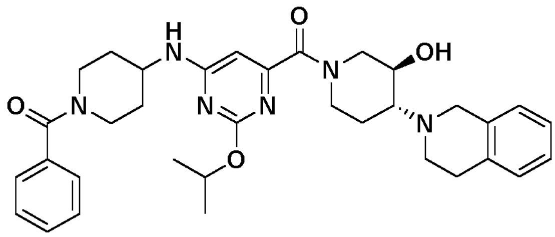
;

in the *p*-toluenesulfonate, the salt-forming ratio of compound I to *p*-toluenesulfonic acid is 1:2.

**19.** A crystal form A of a *p*-toluenesulfonate of compound I, wherein the structure of compound I is as follows:

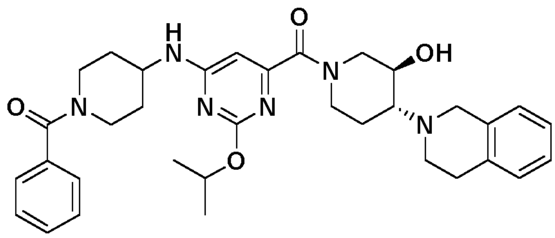
;

in the crystal form A of the *p*-toluenesulfonate, the salt-forming ratio of compound I to *p*-toluenesulfonic acid is 1:2.

**20.** The crystal form A of the *p*-toluenesulfonate of compound I as claimed in claim 19, wherein

the crystal form A of the *p*-toluenesulfonate of compound I has characteristic peaks at 2θ angles of 4.0±0.2°, 8.1 ±0.2°, 18.3±0.2°, and 20.0±0.2° in an XRPD pattern using Cu-Kα radiation;
preferably, the crystal form A of the *p*-toluenesulfonate of compound I has characteristic peaks at 2θ angles of 4.0 ±0.2°, 8.1±0.2°, 11.7±0.2°, 12.7±0.2°, 14.6±0.2°, 18.3±0.2°, 20.0±0.2°, and 23.1±0.2° in the XRPD pattern using Cu-Kα radiation;
more preferably, the crystal form A of the *p*-toluenesulfonate of compound I has characteristic peaks at 2θ angles of 4.0±0.2°, 6.2±0.2°, 8.1±0.2°, 8.8±0.2°, 11.7±0.2°, 12.7±0.2°, 14.6±0.2°, 16.3±0.2°, 17.5±0.2°, 18.3 ±0.2°, 19.0±0.2°, 20.0±0.2°, and 23.1±0.2° in the XRPD pattern using Cu-Kα radiation;
further preferably, the crystal form A of the p-toluenesulfonate of compound I has an XRPD pattern substantially as shown in FIG. 10.

**21.** The crystal form A of the p-toluenesulfonate of compound I as claimed in claim 19 or 20, wherein

a DSC profile of the crystal form A of the p-toluenesulfonate of compound I has endothermic peaks at around 88.5 °C and around 150.1 °C;
preferably, the DSC profile of the crystal form A of the p-toluenesulfonate of compound I has endothermic peaks at 88.5 °C ± 5 °C and 150.1 °C ± 5 °C;
more preferably, the crystal form A of the p-toluenesulfonate of compound I has a DSC profile substantially as shown in FIG. 11.

**22.** The crystal form A of the p-toluenesulfonate of compound I as claimed in any one of claims 19 to 21, wherein

a TGA profile of the crystal form A of the p-toluenesulfonate of compound I shows a weight loss of about 5.7% at around 130.0 °C, and also shows a weight loss of about 4.7% at around 220.0 °C;
preferably, the TGA profile of the crystal form A of the p-toluenesulfonate of compound I shows a weight loss of about 5.7% at 130.0 °C ± 5 °C, and also shows a weight loss of about 4.7% at 220.0 °C ± 5 °C;
more preferably, the crystal form A of the p-toluenesulfonate of compound I has a TGA profile substantially as shown in FIG. 11.

**23.** A crystal form B of a *p*-toluenesulfonate of compound I, wherein the structure of compound I is as follows:

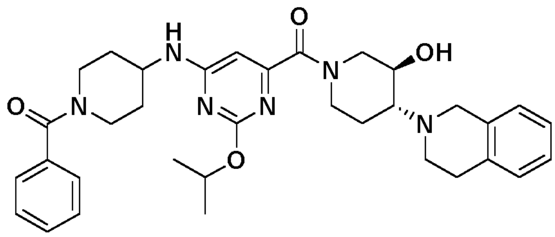

;

in the crystal form B of the *p*-toluenesulfonate, the salt-forming ratio of compound I to *p*-toluenesulfonic acid is 1:2.

**24.** The crystal form B of the p-toluenesulfonate of compound I as claimed in claim 23, wherein

the crystal form B of the p-toluenesulfonate of compound I has characteristic peaks at 2θ angles of 4.3±0.2°, 19.1 ±0.2°, and 20.2±0.2° in an XRPD pattern using Cu-Kα radiation; preferably, the crystal form B of the p-toluenesulfonate of compound I has characteristic peaks at 2θ angles of 4.3±0.2°, 9.8±0.2°, 11.5±0.2°, 19.1±0.2°, and 20.2±0.2° in the XRPD pattern using Cu-Kα radiation;
more preferably, the crystal form B of the p-toluenesulfonate of compound I has characteristic peaks at 2θ angles of 4.3±0.2°, 4.9±0.2°, 9.8±0.2°, 11.5±0.2°, 17.2±0.2°, 19.1±0.2°, 20.2±0.2°, and 21.5±0.2° in the XRPD pattern using Cu-Kα radiation;
further preferably, the crystal form B of the *p*-toluenesulfonate of compound I has characteristic peaks at 2θ angles of 4.3±0.2°, 4.9±0.2°, 9.0±0.2°, 9.8±0.2°, 11.5±0.2°, 17.2±0.2°, 19.1±0.2°, 20.2±0.2°, 21.5±0.2°, and 25.7 ±0.2° in the XRPD pattern using Cu-Kα radiation;
still further preferably, the crystal form B of the p-toluenesulfonate of the compound I has characteristic peaks at 2θ angles of 4.3±0.2°, 4.9±0.2°, 7.1±0.2°, 9.0±0.2°, 9.8±0.2°, 11.5±0.2°, 12.8±0.2°, 17.2±0.2°, 19.1±0.2°, 20.2 ±0.2°, 21.5±0.2°, and 25.7±0.2° in the XRPD pattern using Cu-Kα radiation;
yet still further preferably, the crystal form B of the p-toluenesulfonate of compound I has an XRPD pattern substantially as shown in FIG. 13 or FIG. 15.

**25.** The crystal form B of the *p*-toluenesulfonate of compound I as claimed in claim 23 or 24, wherein

a DSC profile of the crystal form B of the p-toluenesulfonate of compound I has an endothermic peak at around 192.1 °C, and preferably also has an endothermic peak at around 124.8 °C;
preferably, the DSC profile of the crystal form B of the p-toluenesulfonate of compound I has an endothermic peak at 192.1 °C ± 5 °C, and preferably also has an endothermic peak at 124.8 °C ± 5 °C;
more preferably, the crystal form B of the *p*-toluenesulfonate of compound I has a DSC profile substantially as shown in FIG. 16.

**26.** The crystal form B of the *p*-toluenesulfonate of compound I as claimed in any one of claims 23 to 25, wherein

a TGA profile of the crystal form B of the p-toluenesulfonate of compound I shows a weight loss of about 2.0% at around 167.3 °C, and also shows a weight loss of about 5.2% at around 233.7 °C;
preferably, the TGA profile of the crystal form B of the p-toluenesulfonate of compound I shows a weight loss of about 2.0% at 167.3 °C ± 5 °C, and also shows a weight loss of about 5.2% at 233.7 °C ± 5 °C;
more preferably, the crystal form B of the p-toluenesulfonate of compound I has a TGA profile substantially as shown in FIG. 16.

**27.** The crystal form B of the p-toluenesulfonate of compound I as claimed in any one of claims 23 to 26, wherein the crystal form B of the p-toluenesulfonate of compound I is a hydrate, preferably a monohydrate.

**28.** A pharmaceutical composition, comprising the salt of the compound I or the crystal form thereof as claimed in any one of claims 4 to 27.

**29.** The use of the salt of the compound I or the crystal form thereof as claimed in any one of claims 4 to 27, or the pharmaceutical composition as claimed in claim 28, for the manufacturing of a medicament for the prevention and/or treatment of PRMT5-mediated diseases and/or disorders.

**30.** The use of the salt of the compound I or the crystal form thereof as claimed in any one of claims 4 to 27, or the pharmaceutical composition as claimed in claim 28, for the manufacturing of a medicament for the prevention and/or treatment of tumor diseases; preferably, the tumor diseases are solid tumors or hematological tumors, preferably malignant solid tumors or hematological tumors;
more preferably, the tumor diseases are lymphomas.

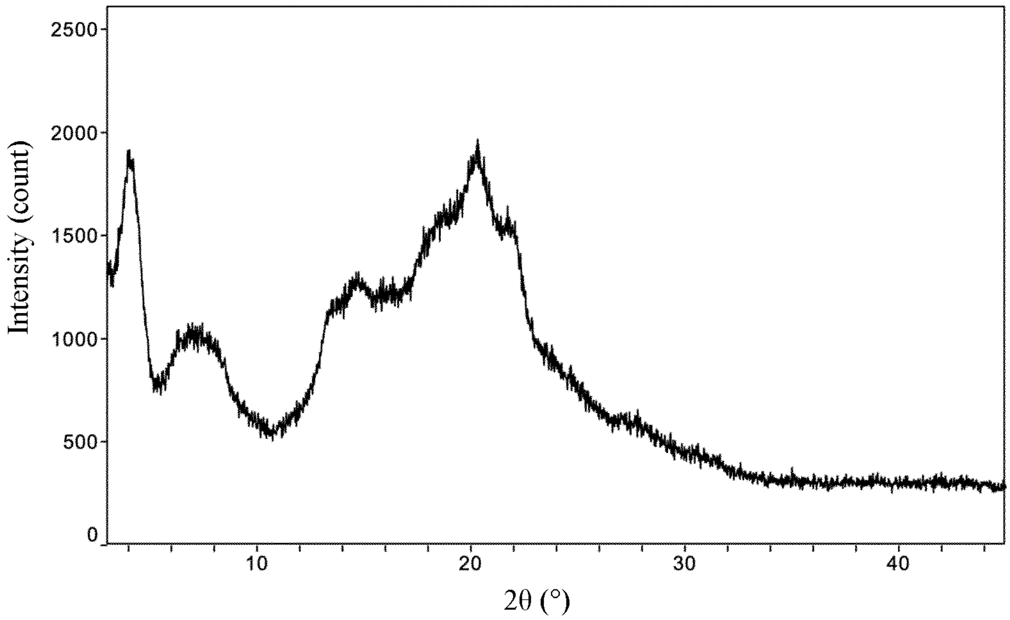
Intensity (count)
2500
2000
1500
1000
500
0
10
20
30
40
2θ (°)

FIG. 1

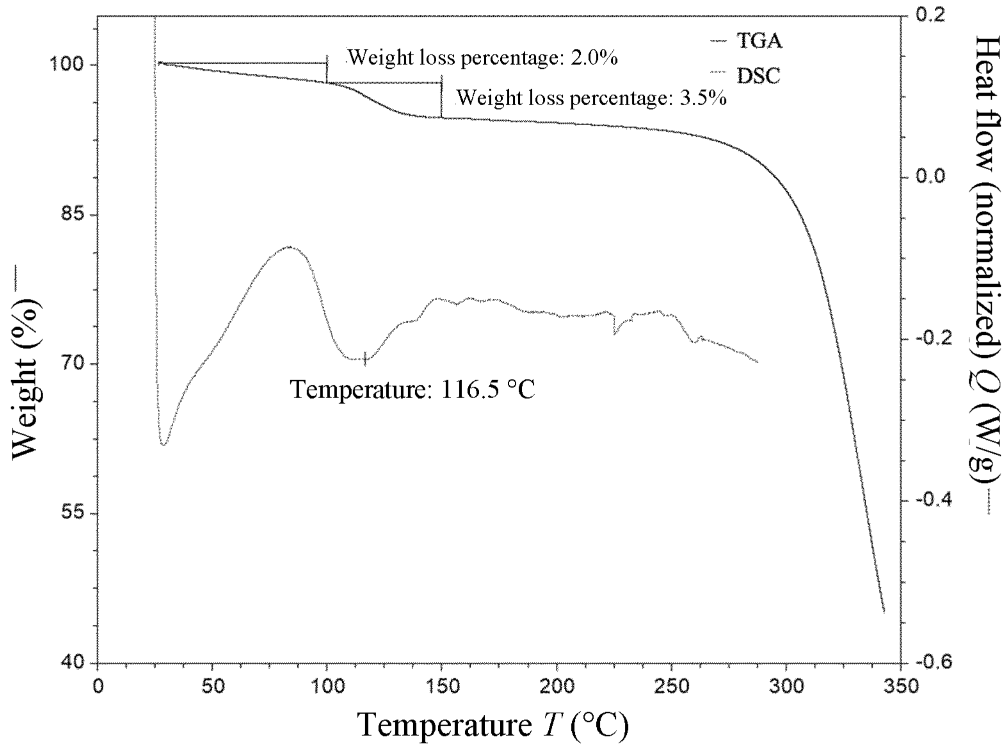
Weight loss percentage: 2.0%
Weight loss percentage: 3.5%
TGA
DSC
Temperature: 116.5 °C
Weight (%)
Heat flow (normalized) Q (W/g)
Temperature T (°C)

FIG. 2

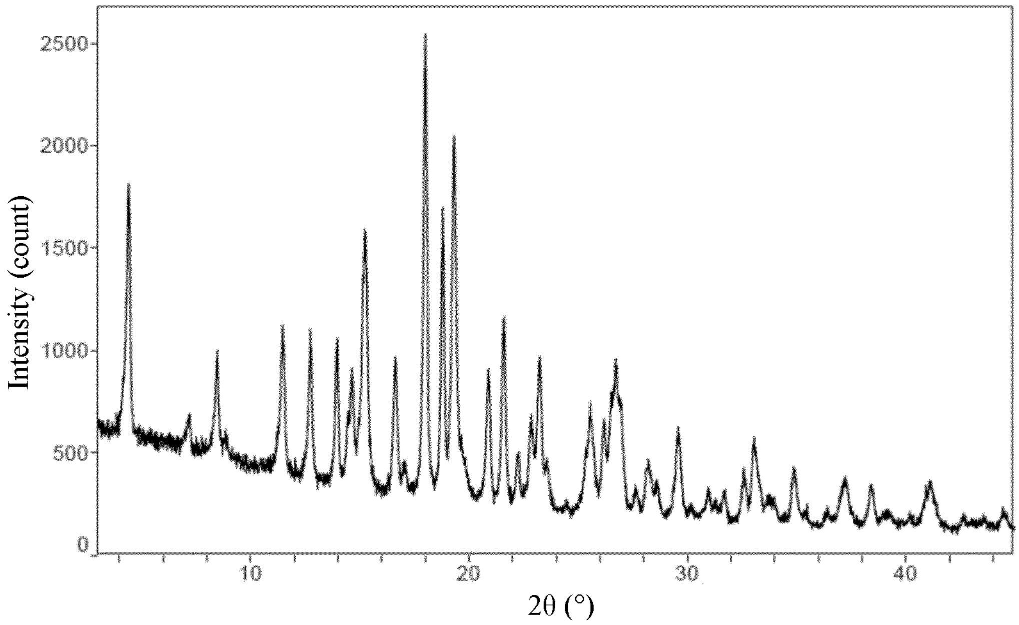
Intensity (count)
2500
2000
1500
1000
500
0
10
20
30
40
2θ (°)

FIG. 3

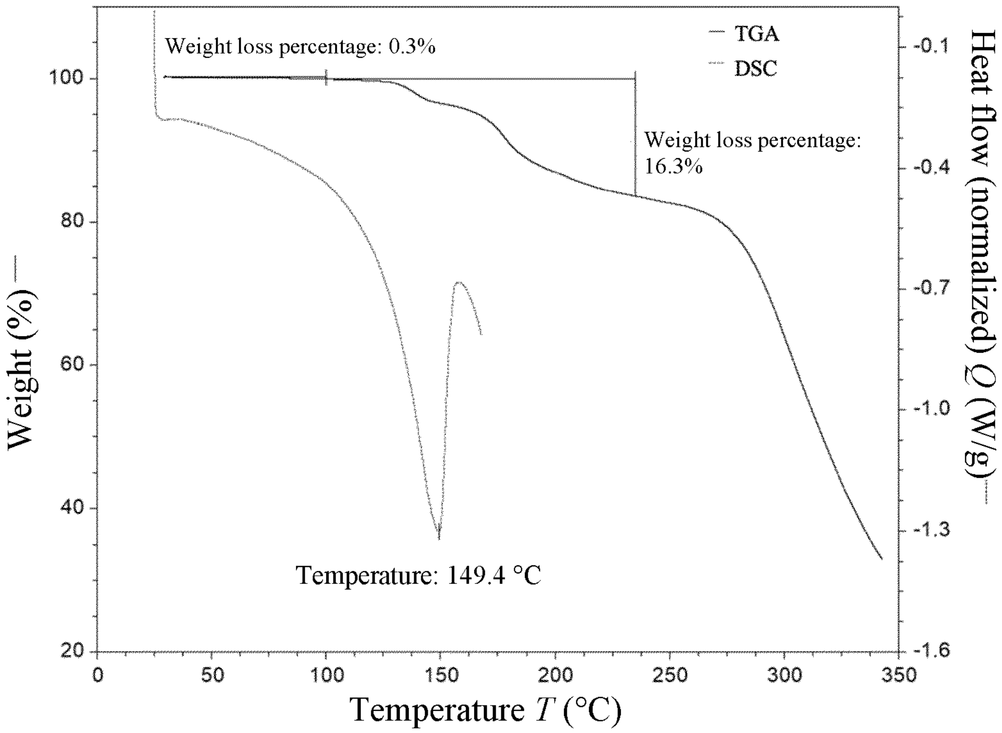
TGA
DSC
Weight loss percentage: 0.3%
Weight loss percentage: 16.3%
Temperature: 149.4 °C
Weight (%)
Heat flow (normalized) Q (W/g)
100
80
60
40
20
-0.1
-0.4
-0.7
-1.0
-1.3
-1.6
0
50
100
150
200
250
300
350
Temperature T (°C)

FIG. 4

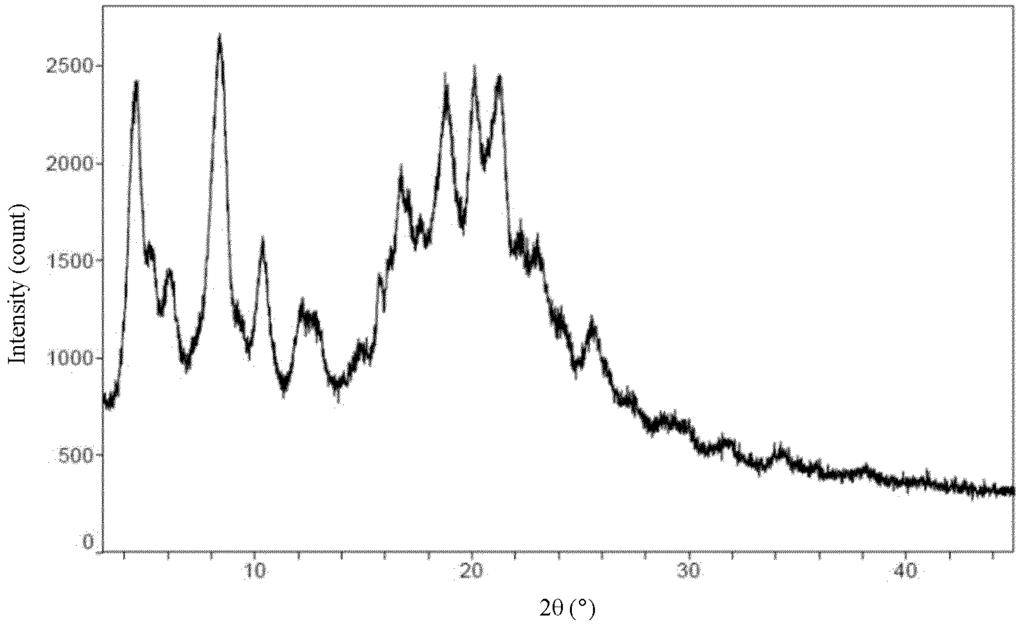
Intensity (count)
2θ (°)

FIG. 5

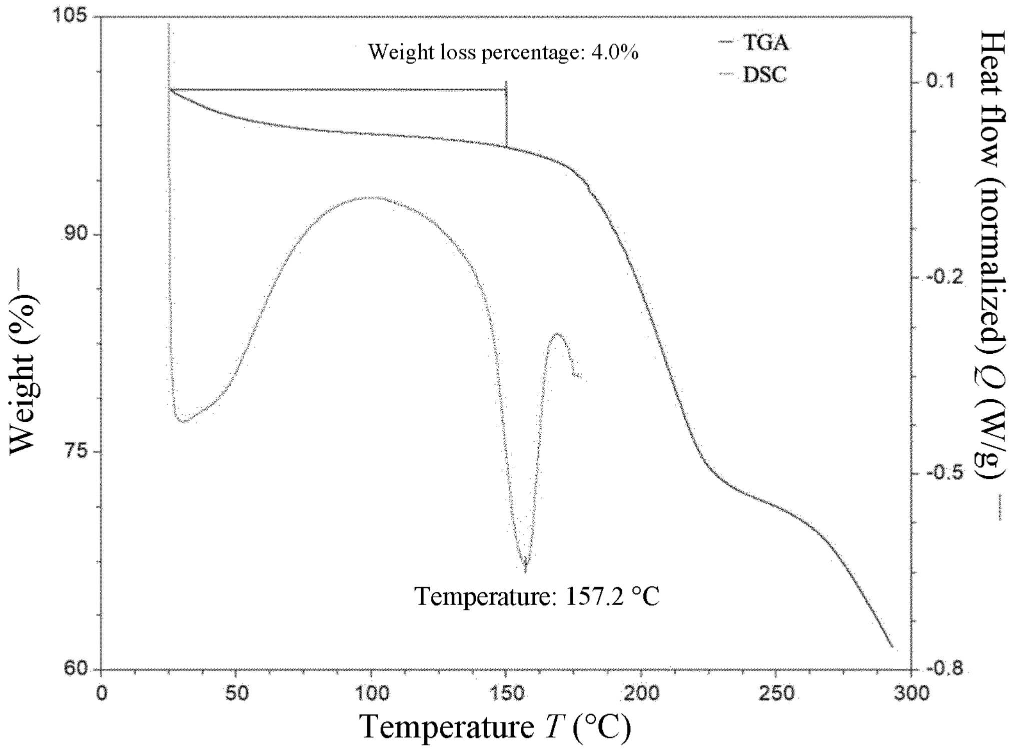
Weight loss percentage: 4.0%
TGA
DSC
Temperature: 157.2 °C
Weight (%)
Heat flow (normalized) Q (W/g)
Temperature T (°C)

FIG. 6

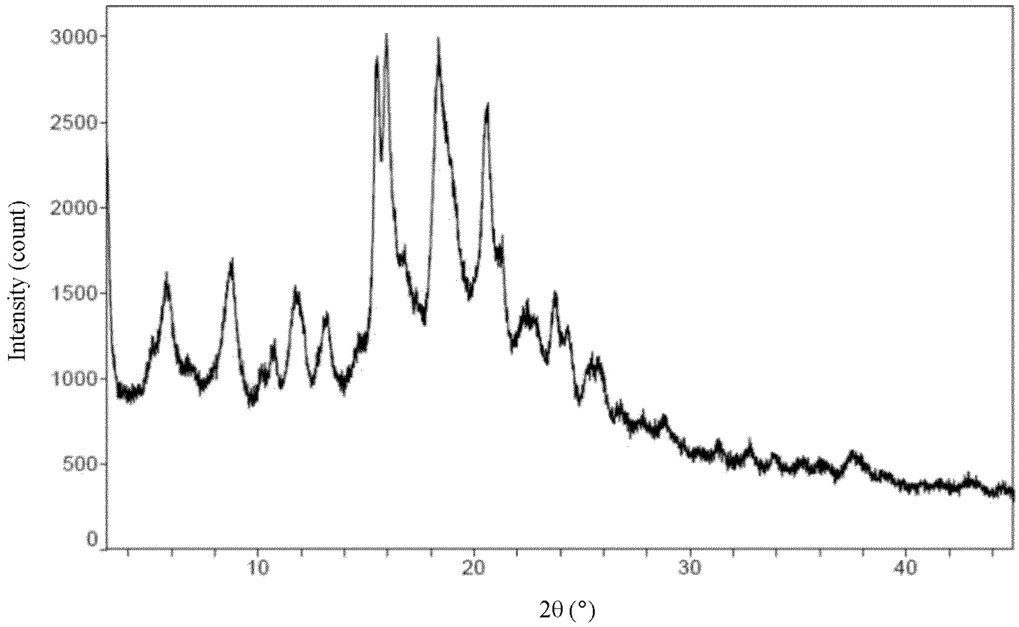
3000
2500
2000
1500
1000
500
0
Intensity (count)
10
20
30
40
2θ (°)

FIG. 7

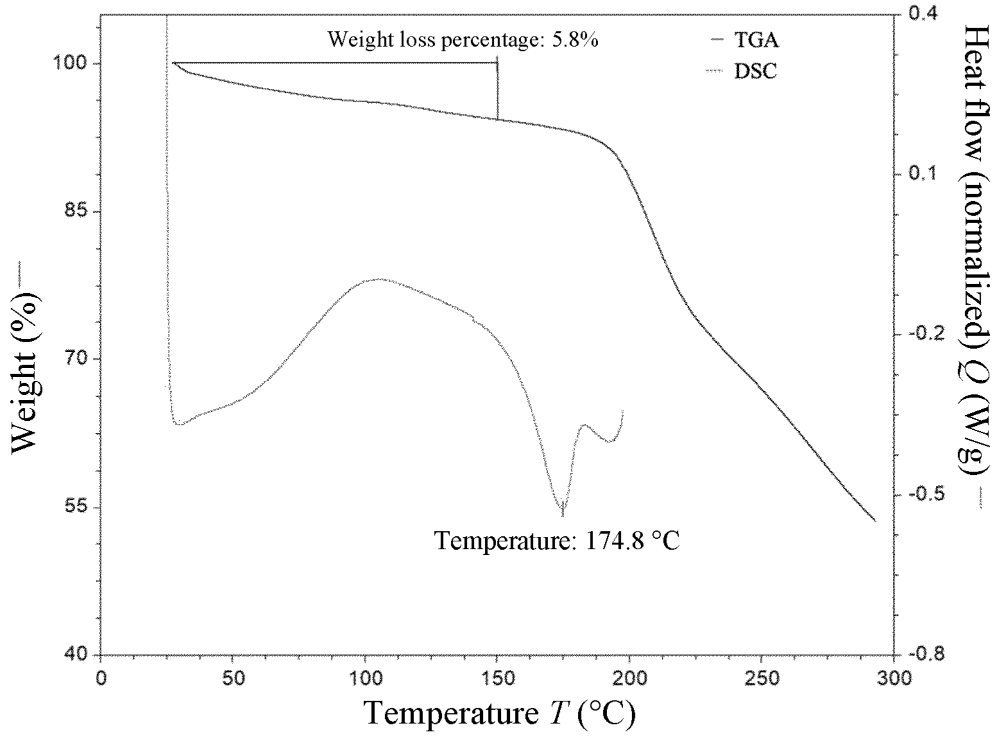
Weight loss percentage: 5.8%
TGA
DSC
100
85
70
55
40
Weight (%)
0.4
0.1
-0.2
-0.5
-0.8
Heat flow (normalized) Q (W/g)
Temperature: 174.8 °C
0
50
100
150
200
250
300
Temperature T (°C)

FIG. 8

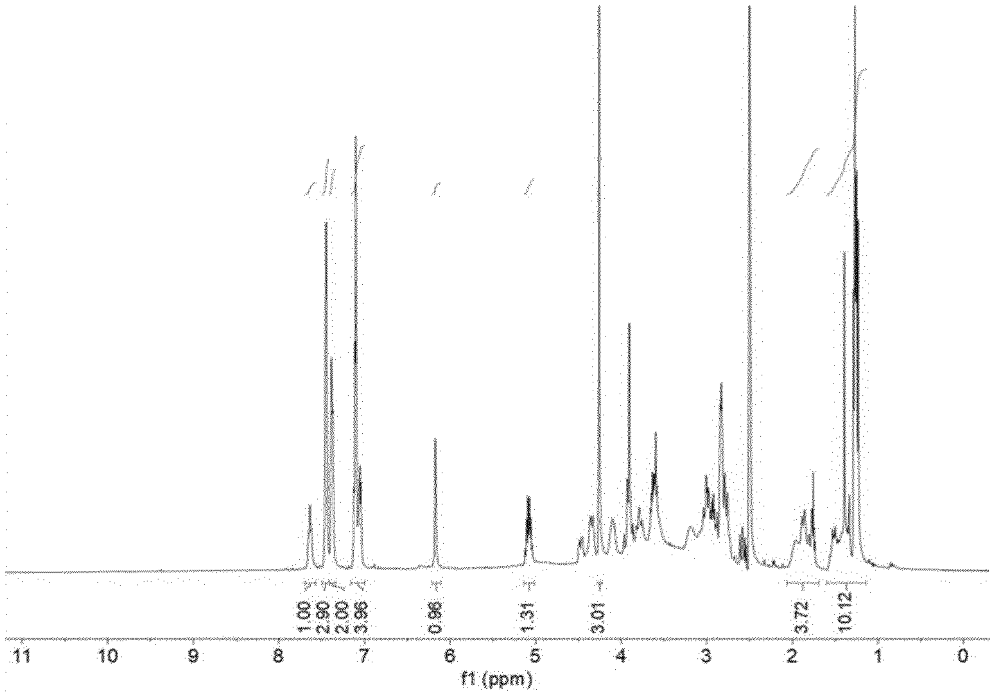
1.00
2.90
2.00
3.96
0.96
1.31
3.01
3.72
10.12
11
10
9
8
7
6
5
4
3
2
1
0
f1 (ppm)

FIG. 9

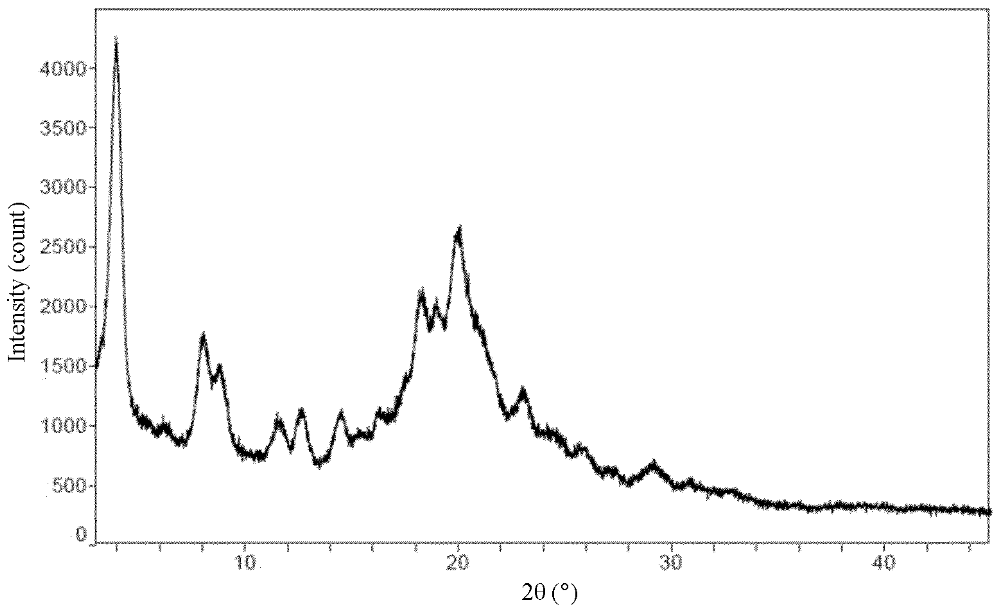
4000
3500
3000
2500
2000
1500
1000
500
0
Intensity (count)
10
20
30
40
2θ (°)

FIG. 10

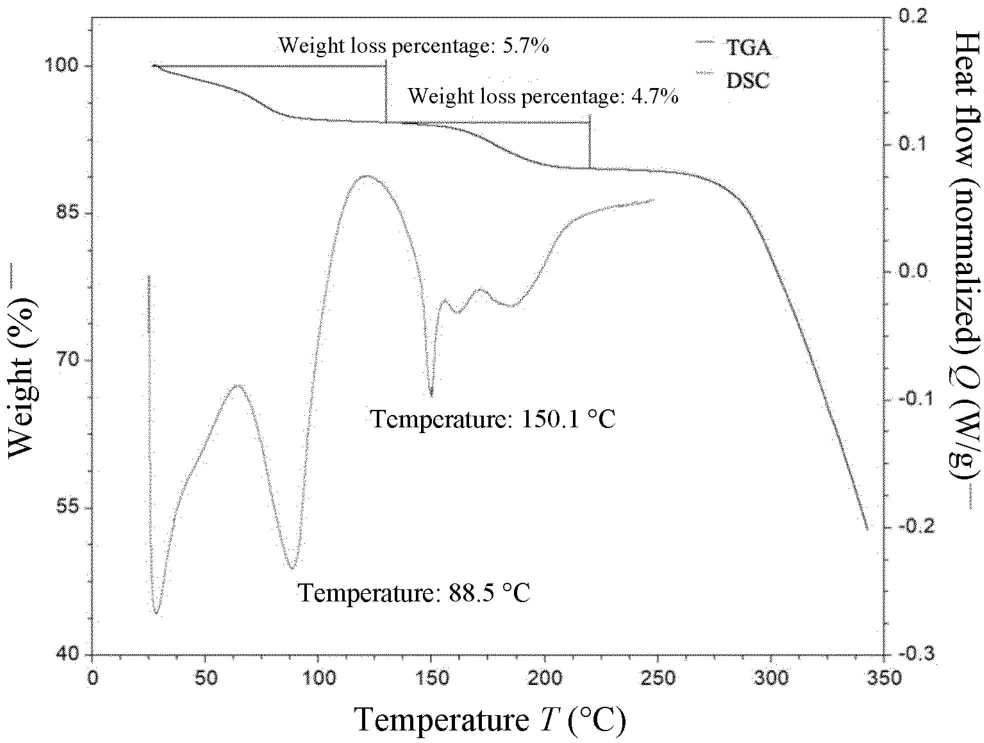
Weight loss percentage: 5.7%
Weight loss percentage: 4.7%
TGA
DSC
Temperature: 150.1 °C
Temperature: 88.5 °C
Weight (%)
Heat flow (normalized) Q (W/g)
Temperature T (°C)
100
85
70
55
40
0.2
0.1
0.0
-0.1
-0.2
-0.3
0
50
100
150
200
250
300
350

FIG. 11

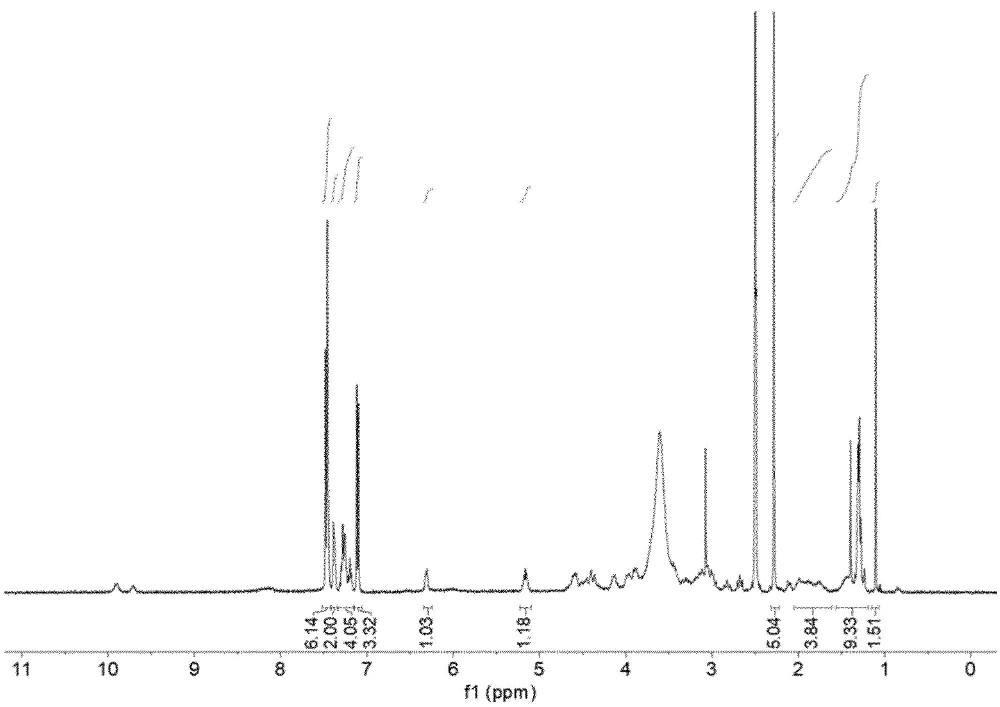
6.14
2.00
4.05
3.32
1.03
1.18
5.04
3.84
9.33
1.51
11
10
9
8
7
6
5
4
3
2
1
0
f1 (ppm)

FIG. 12

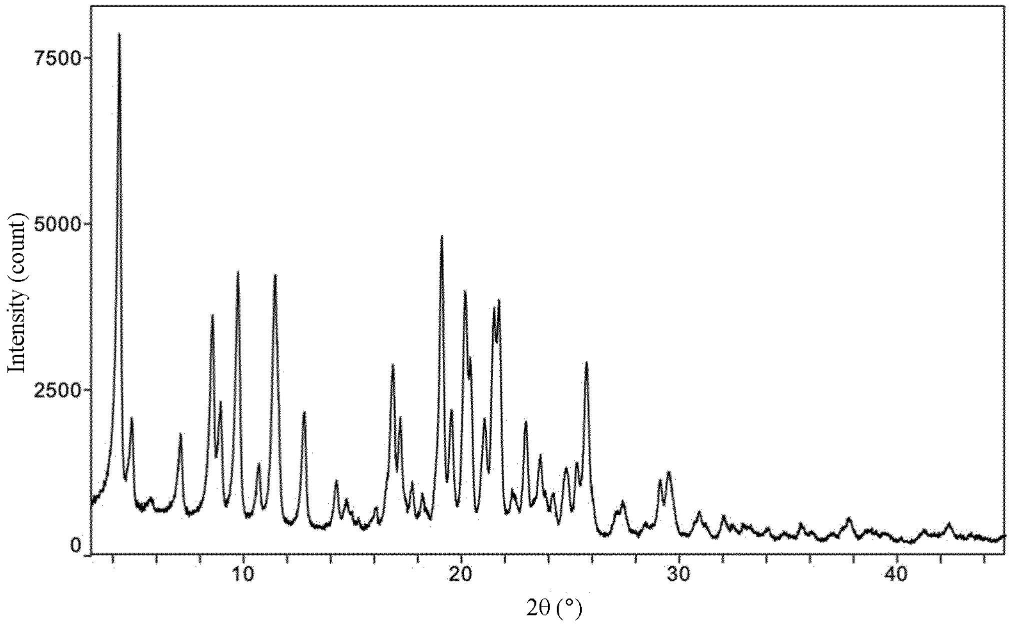
7500
5000
2500
0
Intensity (count)
10
20
30
40
2θ (°)

FIG. 13

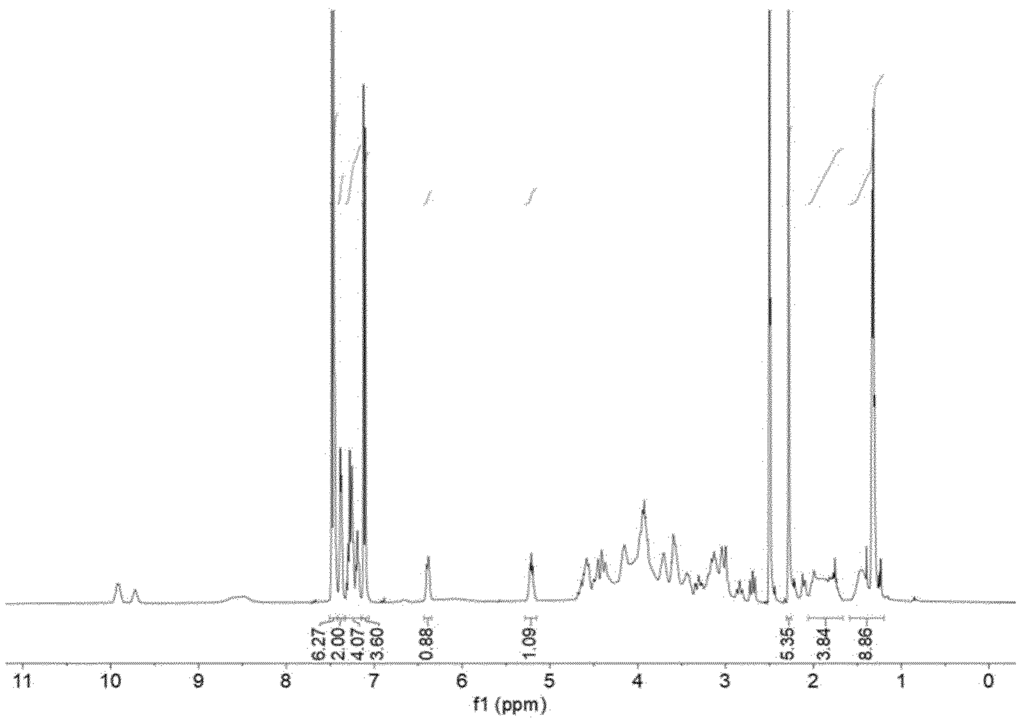
6.27
2.00
4.07
3.60
0.88
1.09
5.35
3.84
8.86
11
10
9
8
7
6
5
4
3
2
1
0
f1 (ppm)

FIG. 14

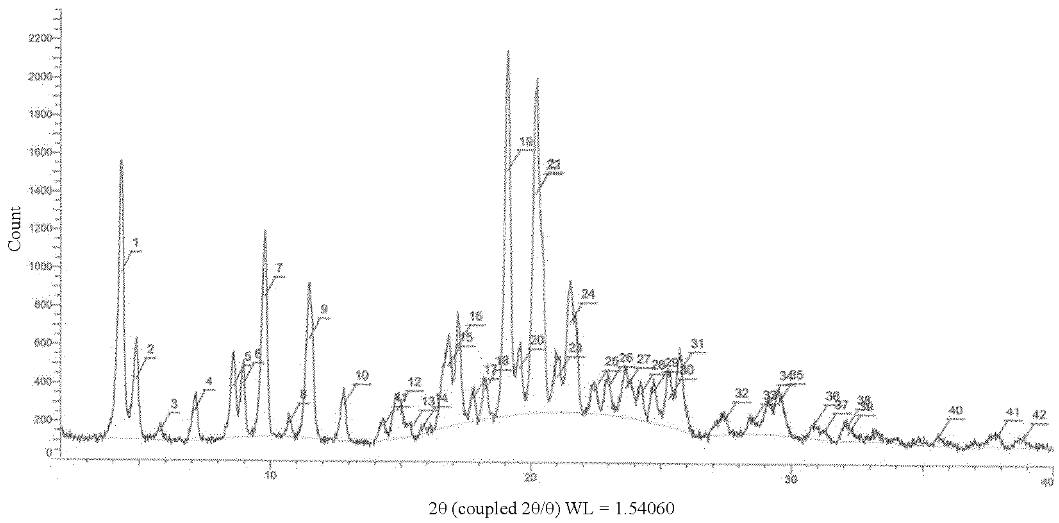
Count
2θ (coupled 2θ/θ) WL = 1.54060

FIG. 15

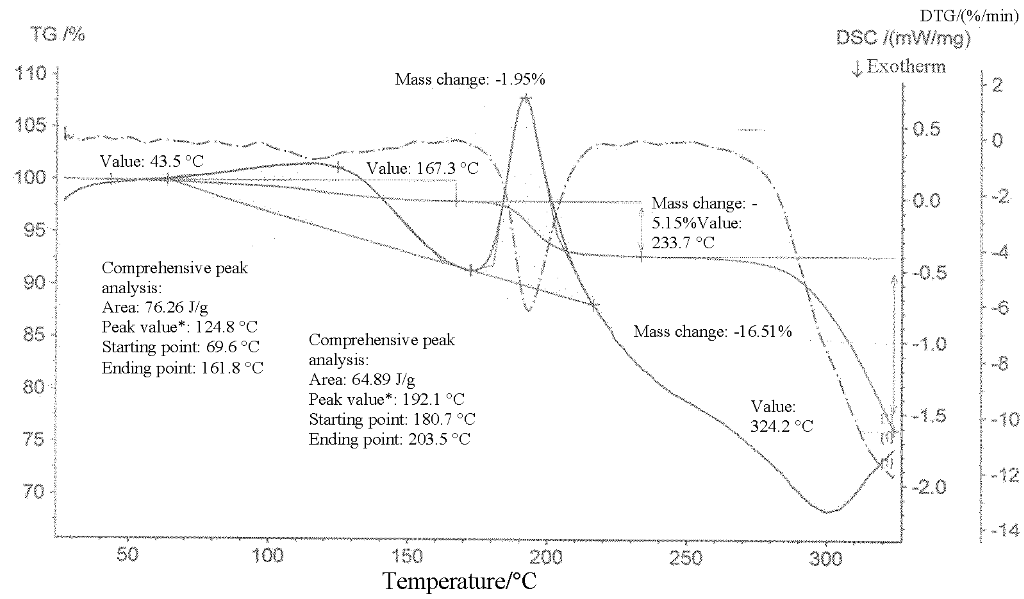
TG /%
DTG/(%/min)
DSC /(mW/mg)
↓ Exotherm
Mass change: -1.95%
Value: 43.5 °C
Value: 167.3 °C
Mass change: -5.15%Value: 233.7 °C
Comprehensive peak analysis:
Area: 76.26 J/g
Peak value*: 124.8 °C
Starting point: 69.6 °C
Ending point: 161.8 °C
Comprehensive peak analysis:
Area: 64.89 J/g
Peak value*: 192.1 °C
Starting point: 180.7 °C
Ending point: 203.5 °C
Mass change: -16.51%
Value: 324.2 °C
Temperature/°C

FIG. 16

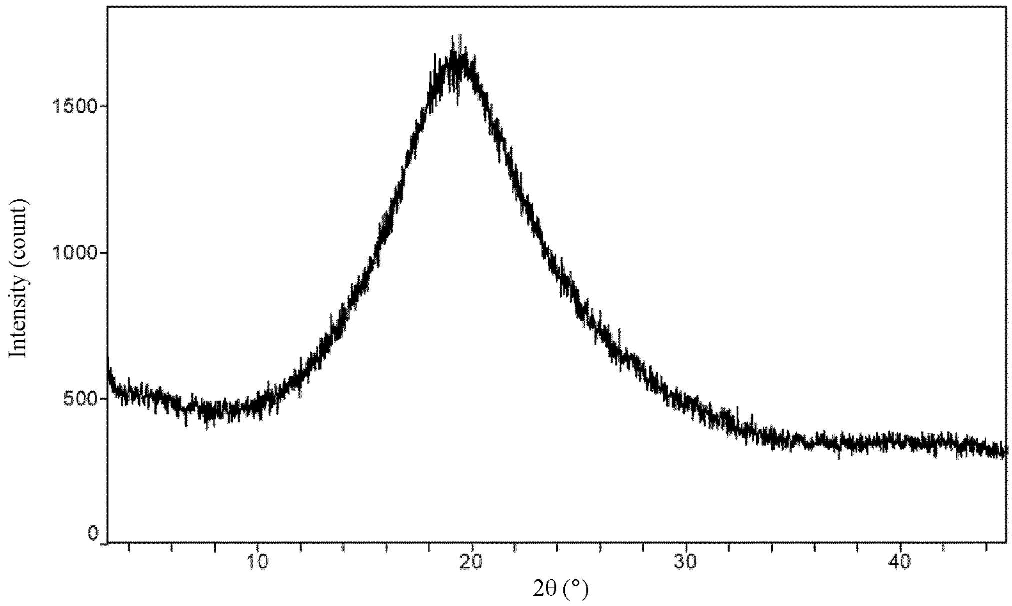


FIG. 17

## INTERNATIONAL SEARCH REPORT

International application No.
**PCT/CN2023/130877**

### A. CLASSIFICATION OF SUBJECT MATTER

C07D401/14(2006.01)i； C07D217/00(2006.01)i； A61K31/4725(2006.01)i； A61P35/00(2006.01)i

According to International Patent Classification (IPC) or to both national classification and IPC

### B. FIELDS SEARCHED

Minimum documentation searched (classification system followed by classification symbols)

IPC:C07D; A61K; A61P

Documentation searched other than minimum documentation to the extent that such documents are included in the fields searched

Electronic data base consulted during the international search (name of data base and, where practicable, search terms used)

DWPI, CNTXT, CNKI, REGISTRY(STN), CAPLUS(STN): 中奇制药, 四氢异喹啉, 哌啶, 嘧啶, 蛋白质精氨酸甲基转移酶, 抑制剂, 癌症, 肿瘤, 晶体, 晶型, PRMT?, tetrahydroisoquinolinyl, piperidinyl, pyrimidinyl, protein arginine methyltransferase, inhibit+, cancer, tumor, tumour, crystal?

### C. DOCUMENTS CONSIDERED TO BE RELEVANT

| Category* | Citation of document, with indication, where appropriate, of the relevant passages | Relevant to claim No. |
|---|---|---|
| PX | WO 2022237858 A1 (INNOVSTONE THERAPEUTICS LIMITED) 17 November 2022 (2022-11-17)<br>description, pages 1-3 and 11-30 | 1-30 |
| X | WO 2022048631 A1 (INNOVSTONE THERAPEUTICS LIMITED) 10 March 2022 (2022-03-10)<br>description, pages 3-5, 14-26 and 29-30, and claims 45-46 | 1-30 |
| X | WO 2020182018 A1 (SICHUAN KELUN-BIOTECH BIOPHARMACEUTICAL CO., LTD.) 17 September 2020 (2020-09-17)<br>description, pages 2-4 and 11-12, and claims 18-22 | 1-30 |
| A | WO 2021126728 A1 (MERCK SHARP & DOHME CORP. et al.) 24 June 2021 (2021-06-24)<br>description, pages 3-6 and 119-145 | 1-30 |
| A | CN 115087638 A (MERCK SHARP & DOHME CORP.) 20 September 2022 (2022-09-20)<br>claims 1-17 | 1-30 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **09 January 2024** | **23 January 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**
**Information on patent family members**

International application No.
**PCT/CN2023/130877**

| Patent document cited in search report | Publication date (day/month/year) | Patent family member(s) | Publication date (day/month/year) |
|---|---|---|---|
| WO 2022237858 A1 | 17 November 2022 | None | |
| WO 2022048631 A1 | 10 March 2022 | JP 2023540548 A | 25 September 2023 |
| | | KR 20230047458 A | 07 April 2023 |
| | | EP 4209485 A1 | 12 July 2023 |
| WO 2020182018 A1 | 17 September 2020 | None | |
| WO 2021126728 A1 | 24 June 2021 | None | |
| CN 115087638 A | 20 September 2022 | EP 4077282 A1 | 26 October 2022 |
| | | EP 4077282 A4 | 08 November 2023 |
| | | BR 112022012032 A2 | 06 September 2022 |
| | | KR 20220123229 A | 06 September 2022 |
| | | US 2023108452 A1 | 06 April 2023 |
| | | MX 2022007535 A | 23 September 2022 |
| | | AU 2020408148 A1 | 16 June 2022 |
| | | CA 3160153 A1 | 24 June 2021 |
| | | WO 2021126731 A1 | 24 June 2021 |
| | | JP 2023507634 A | 24 February 2023 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 202211410146 A
- CN 202210517649
- CN 2022092346 W
- WO 2020182018 A1


### Non-patent literature cited in the description


- Simultaneous thermal analyzer NETZSCH STA449F3 (NETZSCH, GER). Thermal Analysis of the Chinese Pharmacopoeia. 2015, vol. IV